# EUROPEAN PATENT SPECIFICATION

(11) **EP 2 899 269 B1**
(45) Date of publication and mention of the grant of the patent: **17.04.2019**
(21) Application number: 13823838.1
(22) Date of filing: 24.07.2013
(51) Int. Cl.: C12N 15/09, C12N 5/10

(54) **METHOD FOR CLONING T CELL RECEPTOR**
VERFAHREN ZUM KLONEN EINES T-ZELL-REZEPTORS
PROCÉDÉ DE CLONAGE DE RÉCEPTEUR DE LYMPHOCYTE T

(30) Priority: 25.07.2012 JP 2012164442
(43) Date of publication of application: 29.07.2015
(73) Proprietor: National University Corporation University Of Toyama, Toyama-shi, Toyama 930-8555 (JP)
(72) Inventor: KISHI, Hiroyuki, Toyama-shi Toyama 930-0194 (JP); MURAGUCHI, Atsushi, Toyama-shi Toyama 930-0194 (JP); KOBAYASHI, Eiji, Toyama-shi Toyama 930-0194 (JP); OZAWA, Tatsuhiko, Toyama-shi Toyama 930-0194 (JP)
(74) Representative: dompatent von Kreisler Selting Werner - Partnerschaft von Patent- und Rechtsanwälten mbB
(86) International application number: PCT/JP2013/070028
(87) International publication number: WO 2014/017533

(56) References cited:
- US-A1- 2004 072 288
- OZAWA ET AL: "Comprehensive analysis of the functional TCR repertoire at the single-cell level", BIOCHEMICAL AND BIOPHYSICAL RESEARCH COMMUNICATIONS, ACADEMIC PRESS INC. ORLANDO, FL, US, vol. 367, no. 4, 11 January 2008 (2008-01-11), pages 820-825, XP022449879, ISSN: 0006-291X, DOI: 10.1016/J.BBRC.2008.01.011
- BIRKHOLZ K ET AL: "A fast and robust method to clone and functionally validate T-cell receptors", JOURNAL OF IMMUNOLOGICAL METHODS, ELSEVIER SCIENCE PUBLISHERS B.V.,AMSTERDAM, NL, vol. 346, no. 1-2, 31 July 2009 (2009-07-31), pages 45-54, XP026219368, ISSN: 0022-1759, DOI: 10.1016/J.JIM.2009.05.001 [retrieved on 2009-05-07]
- Anonymous: "SC World, Inc. Development of immune therapy systems", , 1 January 2009 (2009-01-01), XP055248673, JAPAN Retrieved from the Internet: URL:http://www.near21.jp/en/nprec/company/ e/e47.pdf [retrieved on 2016-02-09]
- Anonymous: "SC World Inc./Introduction", , 1 January 2005 (2005-01-01), XP055248676, Retrieved from the Internet: URL:http://www.scworld.co.jp/english/indus try/index.html [retrieved on 2016-02-09]
- EIJI KOBAYASHI ET AL: "A novel system for cloning human TCRs", ONCOIMMUNOLOGY, vol. 3, no. 1, 1 January 2014 (2014-01-01) , page e27258, XP055248683, DOI: 10.4161/onci.27258
- EIJI KOBAYASHI ET AL: "A new cloning and expression system yields and validates TCRs from blood lymphocytes of patients with cancer within 10 days", NATURE MEDICINE., vol. 19, no. 11, 13 October 2013 (2013-10-13), pages 1542-1546, XP55248685, US ISSN: 1078-8956, DOI: 10.1038/nm.3358
- HIROSHI HAMANA ET AL.: 'Jinsoku na TCR cDNA Cloning System o Mochiita EBV Tokuiteki Tan'itsu T-Saibo no TCRa/P Repertoire Kaiseki' PROCEEDINGS OF THE JAPANESE SOCIETY FOR IMMUNOLOGY vol. 40, 2011, page 45, XP008176503
- MBL KENKYU-YO SHIYAKU ALLERGY & IMMUNOLOGY T-SELECT MHC TETRAMER 2008, page 3, XP055191327
- PAILLOT R. ET AL.: 'Equine interferon gamma synthesis in lymphocytes after in vivo infection and in vitro stimulation with EHV-1.' VACCINE vol. 23, no. 36, 2005, pages 4541 - 4551, XP027651859

### SEQUENCE LISTING

<110> University of Toyama
<120> Cloning Method for T Cell Receptor
<130> 135204H
<150> JP 2012-164442
   <151> 2012-07-25
<160> 17
<170> PatentIn version 3.5
<210> 1
   <211> 9
   <212> PRT
   <213> Artificial sequence
<220>
   <223> Peptide vaccine AFP357
<400> 1
   Glu Tyr Ser Arg Arg His Pro Gln Leu
   1               5
<210> 2
   <211> 9
   <212> PRT
   <213> Artificial sequence
<220>
   <223> Peptide vaccine AFP403
<400> 2
   Glu Tyr Ser Arg Arg His Pro Gln Leu
   1               5
<210> 3
   <211> 9
   <212> PRT
   <213> Epstein-Barr virus (EBV peptide BRLF-1, 198\201'206)
<400> 3
   Thr Tyr Pro Val Leu Glu Glu Met Phe
   1               5
<210> 4
   <211> 9
   <212> PRT
   <213> Epstein-Barr virus (EBV peptide BMLF-1, 320\201'328)
<400> 4
   Asp Tyr Asn Phe Val Lys Gln Leu Phe
   1               5
<210> 5
   <211> 9
   <212> PRT
   <213> Epstein-Barr virus (EBV peptide LMP2, 222\201'230)
<400> 5
   Ile Tyr Val Leu Val Met Leu Val Leu
   1               5
<210> 6
   <211> 9
   <212> PRT
   <213> Epstein-Barr virus (EBV peptide EBNA3A, 246\201'254)
<400> 6
   Arg Tyr Ser Ile Phe Phe Cys Tyr Met
   1               5
<210> 7
   <211> 9
   <212> PRT
   <213> Epstein-Barr virus (EBV peptide EBNA3B, 217\201'225)
<400> 7
   Thr Tyr Ser Ala Gly Ile Val Gln Ile
   1               5
<210> 8
   <211> 20
   <212> DNA
   <213> Artificial sequence
<220>
   <223> PCR primer alpha-RT
<400> 8
   agcagtgttt ggcagctctt 20
<210> 9
   <211> 20
   <212> DNA
   <213> Artificial sequence
<220>
   <223> PCR primer beta1-RT
<400> 9
   ctggcaaaag aagaatgtgt 20
<210> 10
   <211> 20
   <212> DNA
   <213> Artificial sequence
<220>
   <223> PCR primer beta2-RT
<400> 10
   acacagattg ggagcaggta 20
<210> 11
   <211> 91
   <212> DNA
   <213> Artificial sequence
<220>
   <223> PCR primer AP-1
<220>
   <221> misc_feature
   <222> (91)..(91)
   <223> n is a, c, g, t or u
<400> 11
   acagcaggtc agtcaagcag tagcagcagt tcgataactt cgaattctgc agtcgacggt    60
   accgcgggcc cgggatcccc cccccccccd n                                    91
<210> 12
   <211> 20
   <212> DNA
   <213> Artificial sequence
<220>
   <223> PCR primer alpha-lst
<400> 12
   agagggagaa gaggggcaat 20
<210> 13
   <211> 20
   <212> DNA
   <213> Artificial sequence
<220>
   <223> PCR primer beta1-1st
<400> 13
   ccatgacggg ttagaagctc 20
<210> 14
   <211> 21
   <212> DNA
   <213> Artificial sequence
<220>
   <223> PCR primer beta2-1st
<400> 14
   ggatgaagaa tgacctggga t 21
<210> 15
   <211> 21
   <212> DNA
   <213> Artificial sequence
<220>
   <223> nested PCR primer AP-2
<400> 15
   ggatgaagaa tgacctggga t 21
<210> 16
   <211> 21
   <212> DNA
   <213> Artificial sequence
<220>
   <223> nested PCR primer alpha-Nest
<400> 16
   ggtgaatagg cagacagact t 21
<210> 17
   <211> 20
   <212> DNA
   <213> Artificial sequence
<220>
   <223> nested PCR primer beta-Nest
<400> 17
   gtggccaggc acaccagtgt 20

## Description

### Technical Field

The present invention relates to a method for quickly cloning a T cell receptor (TCR). The present invention is useful in the fields of analysis of T cells, analysis of efficacy of drugs such as peptide vaccines, diagnosis and treatment of diseases, and so forth.

### Background Art

In the T cell receptor (TCR) gene therapy, of which application to specific cancers is mainly investigated, a gene of cancer antigen-specific TCR is introduced into lymphocytes of a cancer patient. The transgenic lymphocytes are cultured in a large quantity, and then returned to the cancer patient. Since TCRs that recognize a tumor antigen peptide are expressed on the lymphocytes, it can be expected that they recognize cancer cells presenting the tumor antigen to specifically attack them, and eventually extinguish the cancer cells.

In order to obtain a gene of an antigen-specific TCR for use in gene therapy, it is necessary to specify a T cell that can recognize a cancer antigen among T cells included in peripheral blood lymphocytes (PBLs) collected from the patient, and clone the TCR gene. The approach generally performed for this purpose comprises establishment of an antigen-specific T cell clone, and this usually requires several months.

Meanwhile, many researches have been conducted about TCR repertoires of antigen-specific T cells. These researches have been conducted by using analytical methods generally performed, for example, a method based on FACS using a panel of monoclonal antibodies (mAbs) directed to a product of a gene of the TCRβ (TRB) V gene family (Non-patent document 1), and a method based on PCR using a panel of TRBV specific primers (Non-patent documents 2 to 4). However, the conventional repertoire analyses did not analyze both TCRα (TRA) V and TRBV, and thus they are imperfect as analysis of the TCR repertoires.

Further, there is raised apprehension that these methods including establishment of a T cell clone may cause a bias in TCR repertoire (Non-patent documents 5 and 6). That is, easily growable T cells will increase in the step of establishing a T cell clone, and amplification efficiency may change depending on used primers in the analysis using PCR.

Further, the group of the inventors of the present invention and other groups reported single cell RT-PCR protocols that enable simultaneous identification of transcription products of CDR3α and CDR3β of TCRs of human (Non-patent document 7) and mouse (Non-patent document 8). However, in these single cell RT-PCR protocols, antigenic specificity of any TCR α/β cDNA pair including a complete translation region of protein has not been determined by cloning it and expressing it in a cell.

### Prior art references

### Non-patent documents

Non-patent document 1: Bieganowska, K. et al., Direct analysis of viral-specific CD8+ T cells with soluble HLA-A2/Tax11-19 tetramer complexes in patients with human T cell lymphotropic virus-associated myelopathy, J. Immunol., 162, 1765-1771 (1999)
Non-patent document 2: Hara, H. et al., Detection of human T lymphotrophic virus type 1 (HTLV-I) proviral DNA and analysis of T cell receptor V beta CDR3 sequences in spinal cord lesions of HTLV-I-associated myelopathy/tropical spastic paraparesis, J. Exp. Med., 180, 831-839 (1994) Non-patent document 3: Saito, M. et al., In vivo selection of T-cell receptor junctional region sequences by HLA-A2 human T-cell lymphotropic virus type 1 Tax11-19 peptide complexes, J. Virol., 75, 1065-1071 (2001)
Non-patent document 4: Eiraku, N. et al., Clonal expansion within CD4+ and CD8+ T cell subsets in human T lymphotropic virus type I-infected individuals, J. Immunol., 161, 6674-6680 (1998)
Non-patent document 5: Zhou, J., Dudley, M.E., Rosenberg, S.A. & Robbins, P.F., Selective growth, in vitro and in vivo, of individual T cell clones from tumor-infiltrating lymphocytes obtained from patients with melanoma, J. Immunol., 173, 7622-9 (2004)
Non-patent document 6: Polz, M.F. & Cavanaugh, C.M., Bias in template-to-product ratios in multitemplate PCR, Appl. Environ. Microbiol., 64, 3724-30 (1998)
Non-patent document 7: Ozawa, T., Tajiri, K., Kishi, H. & Muraguchi, A., Comprehensive analysis of the functional TCR repertoire at the single-cell level, Biochem. Biophys. Res. Commun., 367, 820-825 (2008)
Non-patent Document 8: Dash, P. et Al., Paired analysis of TCRalpha and TCRbeta chains at the single-cell level in mice, J. Clin. Invest., 121, 288-295 (2011)
Non-patent document 9: Birkholz et al., Journal of Immunological Methods 346 (2009), pages 45-54.

Patent document US 2004/072288 A1.

### Summary of the Invention

### Object to be Achieved by the Invention

A quick and bias-free cloning system for TCR gene is, if possible, desirable for TCR gene therapy or researches of TCR repertoires.

### Means for Achieving the Object

The inventors of the present invention attempted to establish a TCR cloning system enabling not only analysis of bias-free TCR repertoires, but also collection of antigen-specific TCR α/β cDNA pairs as well as functional evaluation thereof. The inventors of the present invention first attempted to amplify TCR cDNA from a single antigen-specific T cell, which was separated with a cell sorter and individually contained in a tube, by RT-PCR, but it was found that the amplification efficiency of this method was extremely low, and thus cloning by this method was difficult. Then, an antigen-specific T cell was individually put into a tube as in the previous method, stimulated with a stimulant, and then used for RT-PCR, but the low efficiency was not improved. Then, T cells were stimulated as a population with a stimulant, and then the antigen-specific T cells were individually sorted into tubes and used for RT-PCR. As a result, it was surprisingly found that TCR cDNAs could be collected from 70 to 80% of the cells, and accomplished the present invention.

The present invention provides the followings.
[1] A method for producing a gene of T cell receptor (TCR) specific to an antigen A as defined in claim 1.

Further embodiments are defined in the dependent claims.

### Effect of the Invention

According to the present invention, an objective TCR gene can be cloned in a shorter period of time compared with the conventional methods, for example, within about ten days.

The method of the present invention can be designed so as not to substantially comprise any culture step. By the conventional methods comprising a culture step, a ratio of cells that easily proliferate may increase in a population of cells, and as a result, biased repertoire analysis results may be provided. However, according to the present invention, such a problem is ameliorated.

According to the present invention, even a TCR gene of a T cell clone appearing at a comparatively low frequency may be cloned. This characteristic will be advantageous for selection of an objective TCR such as selection of a candidate TCR to be used in TCR gene therapy.

### Brief Description of the Drawings

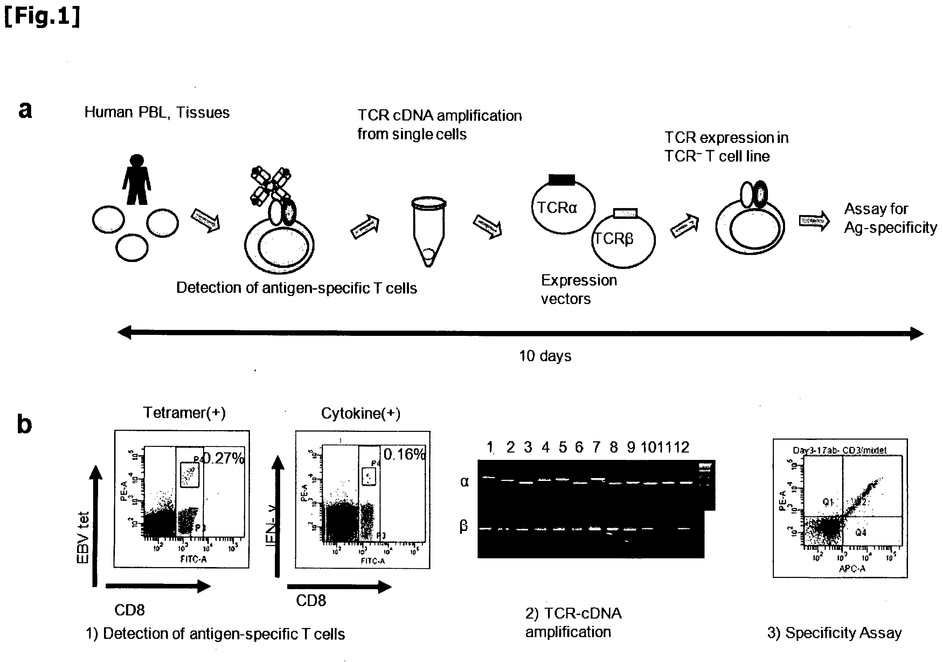

[Fig. 1] Schematic of one embodiment of the present invention, the hTEC10 system. (a) A schematic depicting the procedure of the hTEC10 system. Briefly, by staining T cells contained in human peripheral blood lymphocytes with MHC/peptide (MHC/p) tetramer, antigen-specific T cells are detected, and separated into single cells using a cell sorter. Human TCR cDNAs are amplified from the separated single cells, cloned into an expression vector and then transduced into the TCR-negative T cell line TG40. The antigen-specificity of the TCR is then assessed by staining the TG40 cells with MHC/p tetramers. Alternatively, the antigen-specificity of the expressed TCR can also be assessed by stimulating TG40 cells expressing TCR with an antigen peptide, and analyzing whether the activation marker, CD69, is expressed on the cell surface. According to the present invention, the entire process can be performed within 10 days. (b) Representative data of 1) FACS analysis of antigen-specific T cells (two cases of detection of antigen-specific T cells by using MHC/p tetramers and detection of antigen-specific T cells based on secretion of cytokine), 2) analysis of amplified TCR α/β chain cDNA, and 3) specificity assay using a TCR-transduced T cell line (data obtained by staining TG40 cells expressing TCR with MHC/p tetramer, and analyzing them with a flow cytometer).

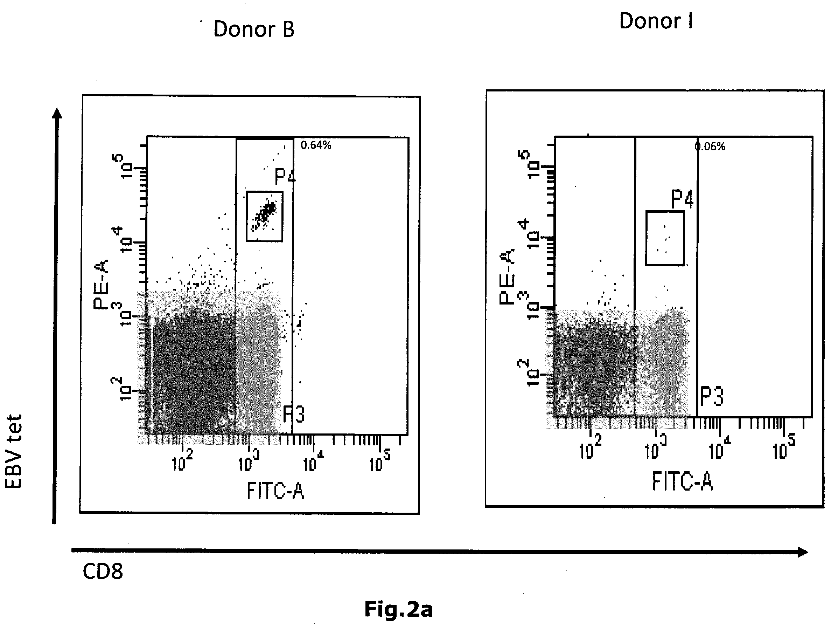

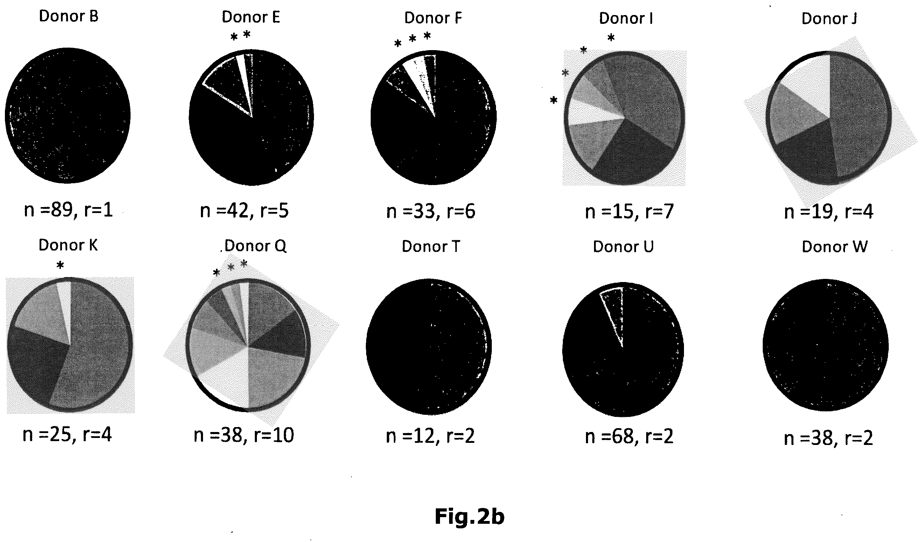

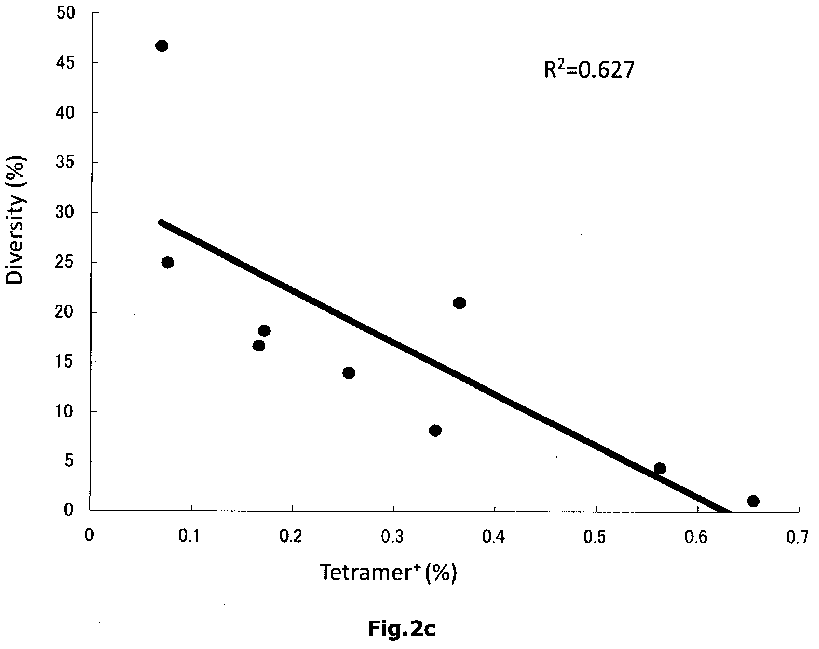

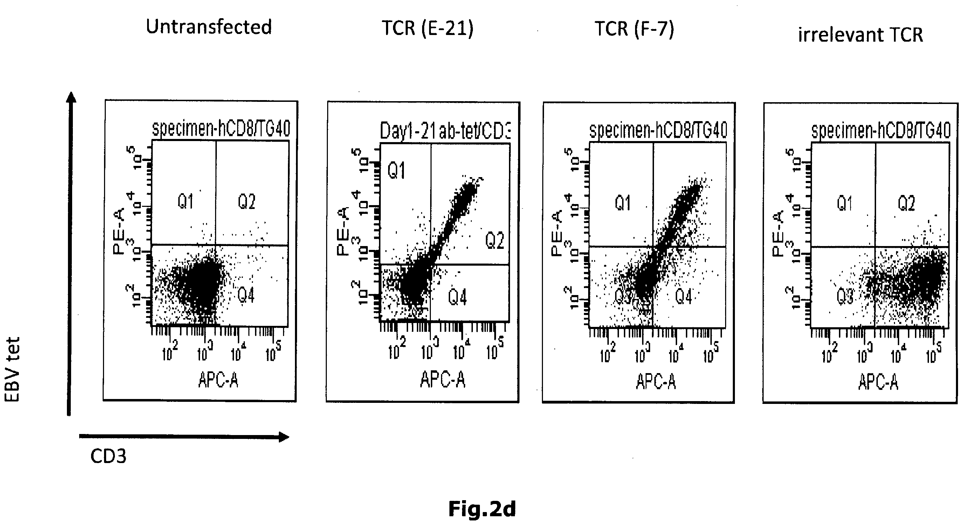

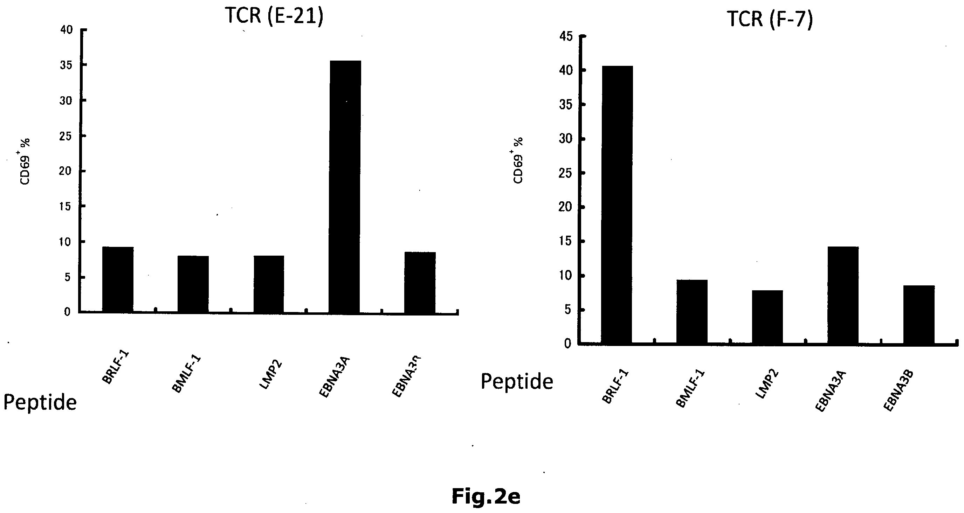

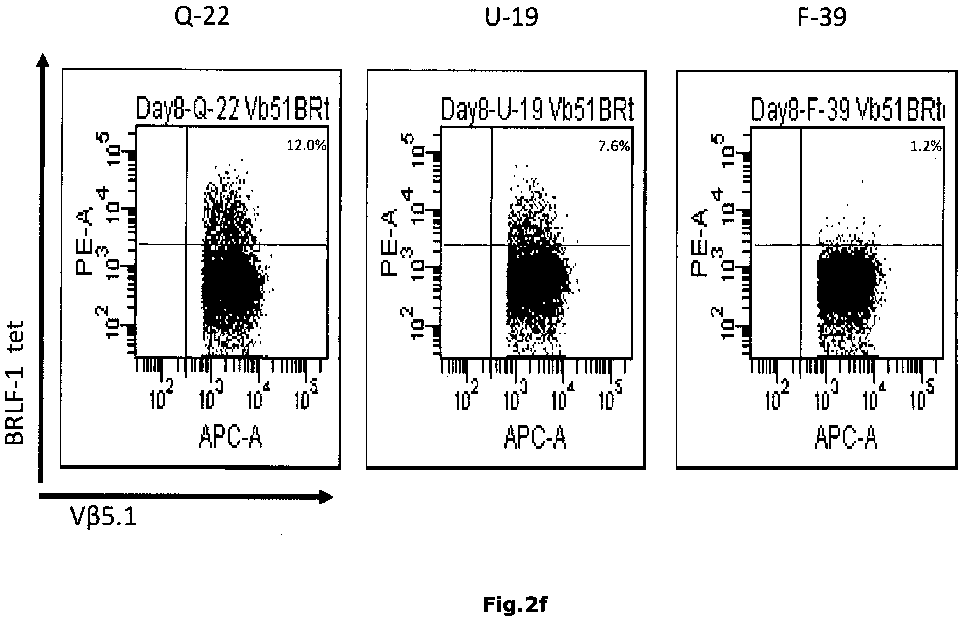

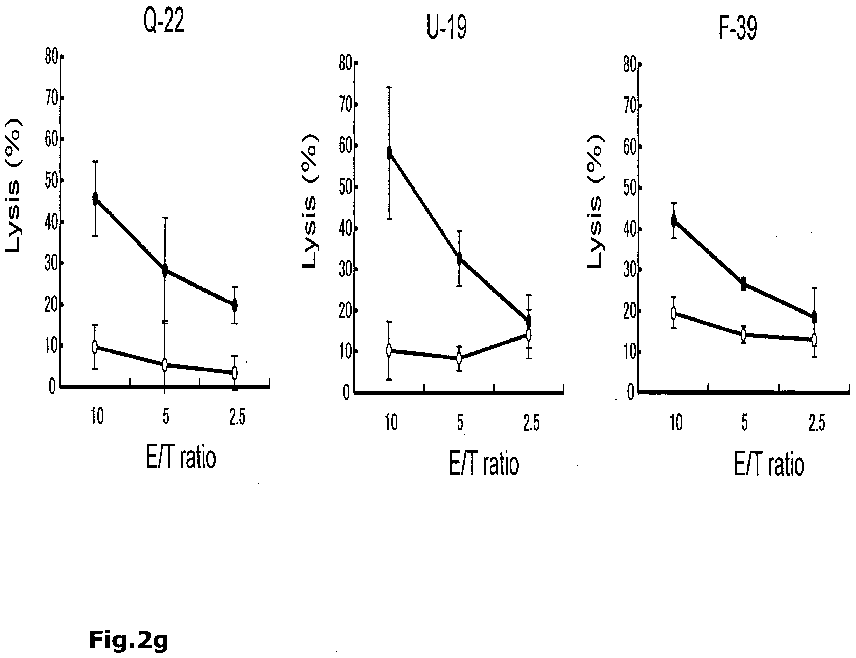

[Fig. 2] Analysis of EBV-specific human TCR α/β pairs obtained by hTEC10. (a) Detection of EB virus (EBV)-specific CD8⁺ T cells in human PBLs. PBLs from 19 HLA-A24⁺ healthy donors were stained with a mixture of an MHC/p tetramer consisting of an anti-CD8 antibody and HLA-A*2402 bound with an EBV-derived peptide (BRLF-1, BMLF-1, LMP-2, EBNA3A, or EBNA3B), and analyzed by using FACS. Representative data of two donors (B and I) are shown. (b) Repertoire analysis of EBV-specific CD8⁺ T cells using sequences of cloned TCR cDNAs from 10 EVB-latent healthy donors. "n" is the number of analyzed T cell clones, "r" represents the repertoire number. An asterisk denotes that the repertoire was obtained from only a single T cell clone. (c) Relationship between clonality and percentage of EBV-specific MHC/p tetramer-positive cells in CD8⁺ T cells of 10 EVB-latent healthy donors. Diversity (%) was calculated using the following formula: Diversity (%) = r/n × 100. "R²" shows the index correlation. (d) Determination of antigen-specificity. TG40 cells were transduced with the indicated cloned TCR and stained with an anti-CD3 antibody and EBV-specific MHC/p tetramer mixture, (e) Enhancement of CD69 expression by antigenic peptide stimulation. E-21 (left) or F-7 (right) TCR-expressing TG40s were incubated with HLA-A24⁺ PBL in the presence of each EBV peptide (BRLF-1 BMLF-1, LMP2, EBNA3A or EBNA3B), and the expression of CD69 was analyzed by FACS. (f) Expression of BRLF-1-specific TCRs on TCR-transduced primary T cells. Primary T cells were retrovirally transduced with three BRLF-1-specific Vβ5.1⁺ TCRs (Q-22, U-19 or F-39), and TCR expression was analyzed by an anti-Vβ5.1 antibody and BRLF-1-specific MHC/p tetramer staining. The value in each profile is the percentage of BRLF-1-specific MHC/p tetramer-positive cells in the Vβ5.1⁺ cell population. (g) Antigen-specific cytotoxicity of cloned TCR-transduced primary T cells. EB virus-specific TCR-transduced primary T cells were incubated with calcein-labeled T2-A24 cells that had been pulsed with BRLF-1 peptide or EBNA3A peptide. The closed and open circles show the cytotoxicity against T2-A24 cells pulsed with BRLF-1 and EBNA3A peptides, respectively. Calcein release from the target cells was measured 4 hours later, and lysis (%) was calculated as described in the Methods section. The results shown are the mean ± SD of triplicate experiments.

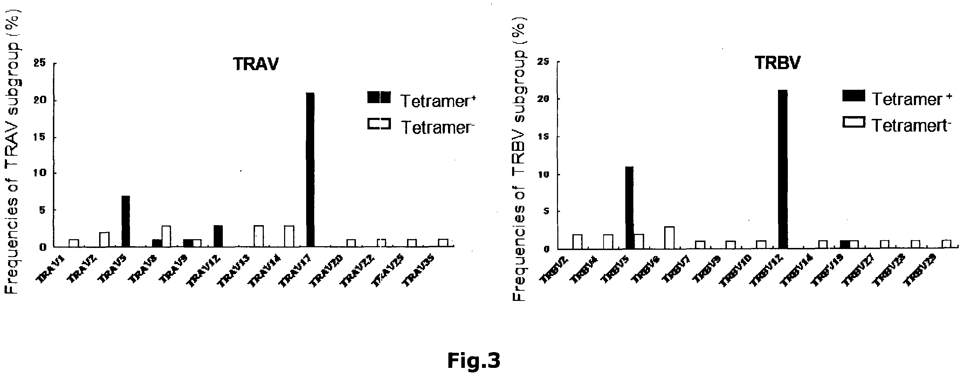

[Fig. 3] No bias in the results obtained by the 5'-RACE single cell PCR method. Repertoire analysis of EBV-specific and BRLF-1-specific MHC/p tetramer positive and negative CD8⁺ T cells. The repertoires of TCR were analyzed by using the IMGT/V-Quest tool (http://www.imgt.org/). Although bias is observed in the repertoire of the EBV-specific MHC/p tetramer positive CD8⁺ T cells for a specific repertoire, no bias was observed in the repertoire of the EBV-specific MHC/p tetramer negative CD8⁺ T cells for a specific repertoire. Therefore, the 5'-RACE single cell PCR method used in this method uniformly amplifies various Vα and Vβ without any bias.

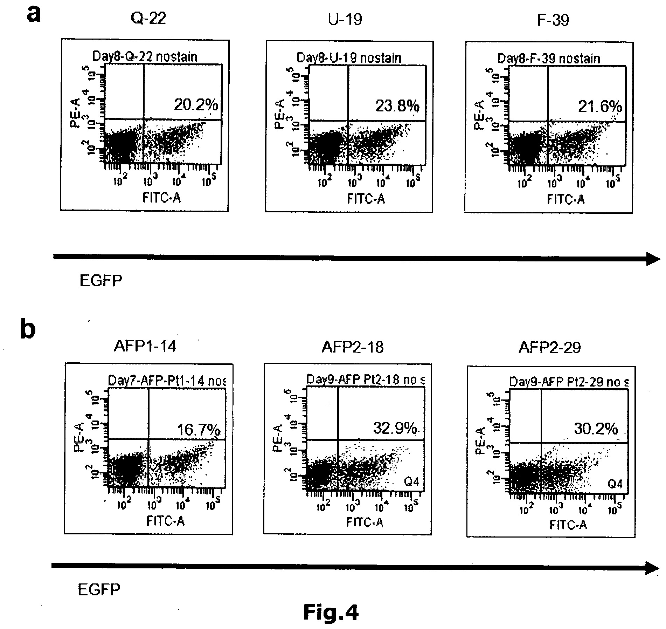

[Fig. 4] Introduction efficiency of the retroviral vector encoding a TCR gene. In order to confirm introduction efficiency into the primary T cells of retroviral vectors encoding the BRLF-1 -specific TCR α/β genes (a), and the AFP-specific TCR α/β genes (b), which were ligated with F2A, expression level of EGFP ligated to the TCR genes via IRES (internal ribosome entry site) was analyzed by FACS.

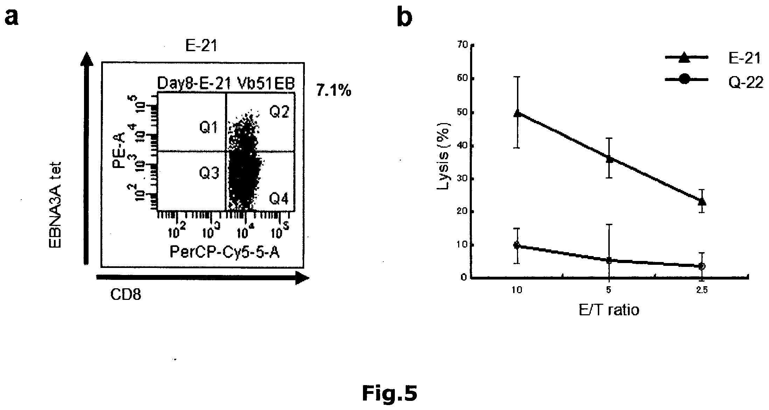

[Fig. 5] Cloned TCR introduced into normal primary T cells gives antigen-specific cytotoxic activity against cells pulsed with the antigen to the T cells into which the cloned TCR is introduced. (a) EBNA3A-specific TCR (E-21) or BRLF-1-specific TCR (Q-22) was retrovirally transduced into activated and proliferating PBL, which was prepared by stimulating PBLs prepared from a healthy donor with CD3CD28 beads over two days. Seven days later, the cells were stained with anti-CD8 antibody and EBNA3A or BRLF-1-specific MHC/p tetramer, and the expression of the introduced TCR was analyzed by flow cytometry. The value mentioned in the drawing is the percentage of the MHC/p tetramer positive cells in the CD8⁺ cell population. (b) Calcein was introduced into the TCR-introduced primary T cells, and the cells were incubated together with T2-A24 cells pulsed with the EBNA3A peptide. Four hours later, release of calcein due to cell injury was measured. The shown results are indicated as average ± SD of the measured values for 3 wells set for each experimental group.

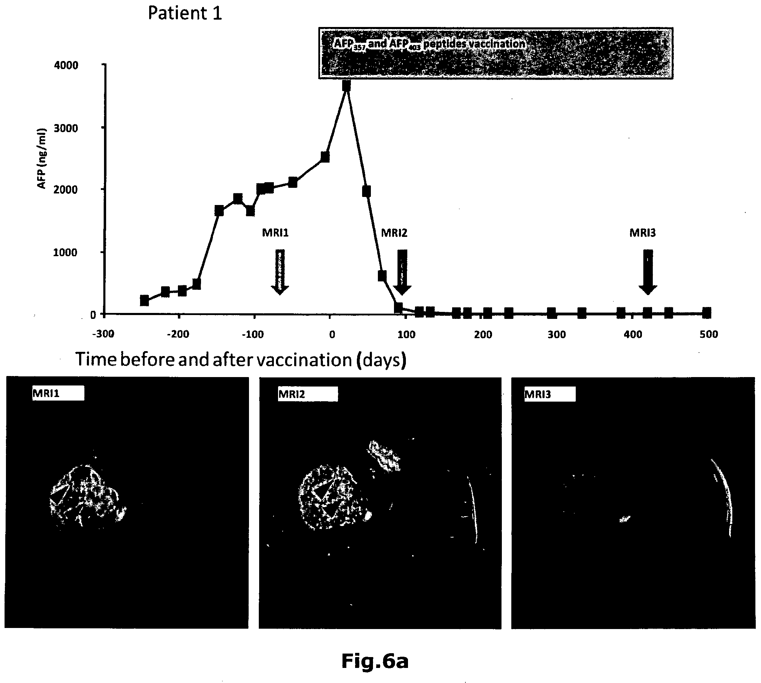

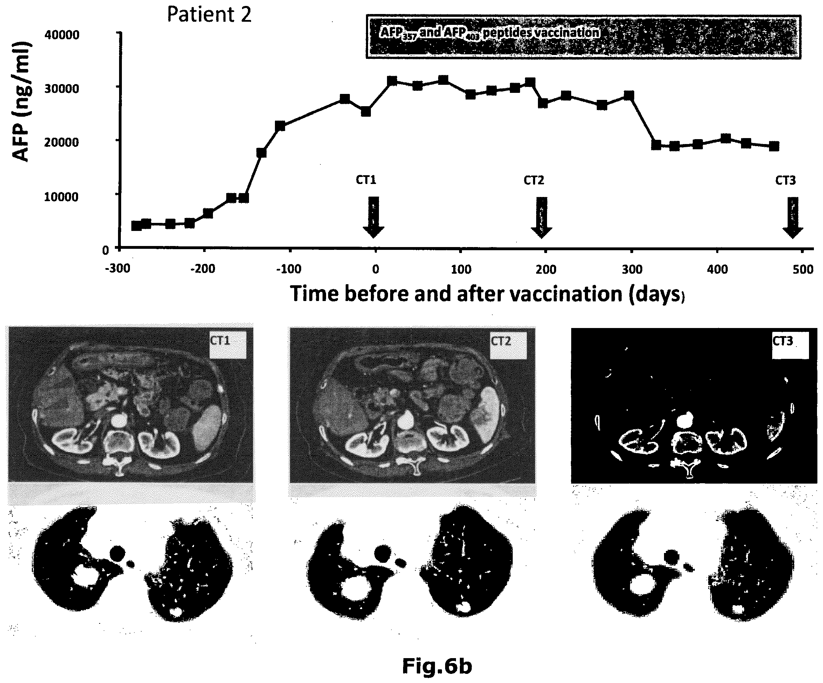

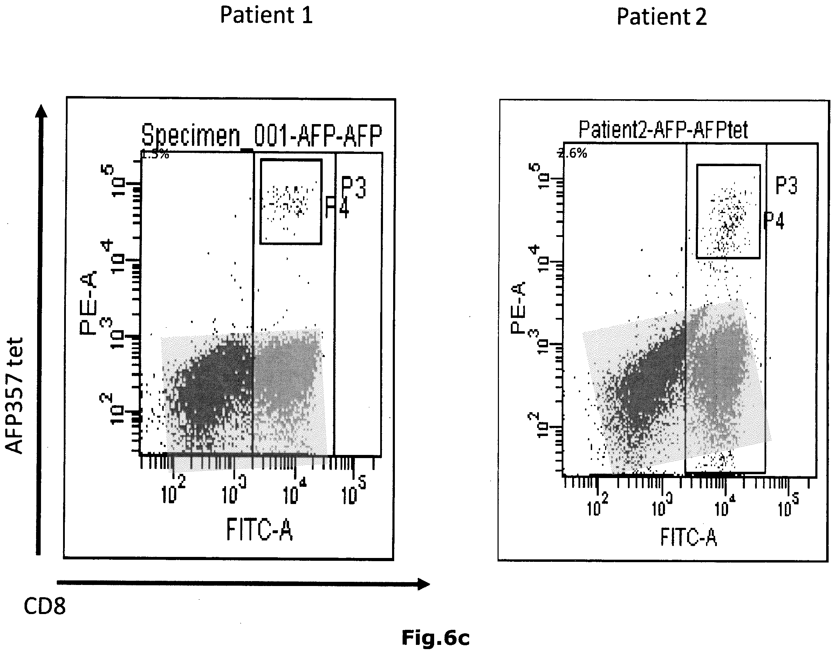

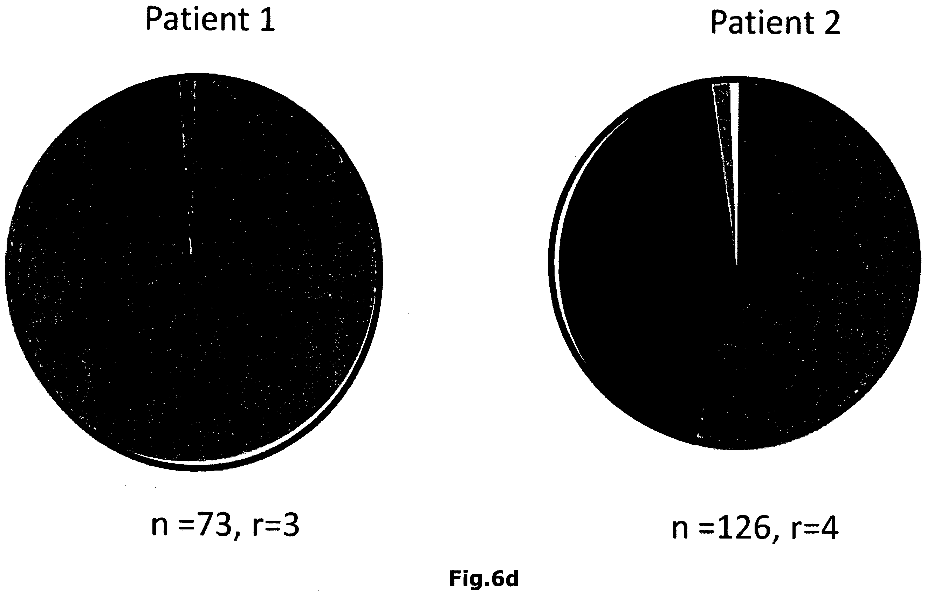

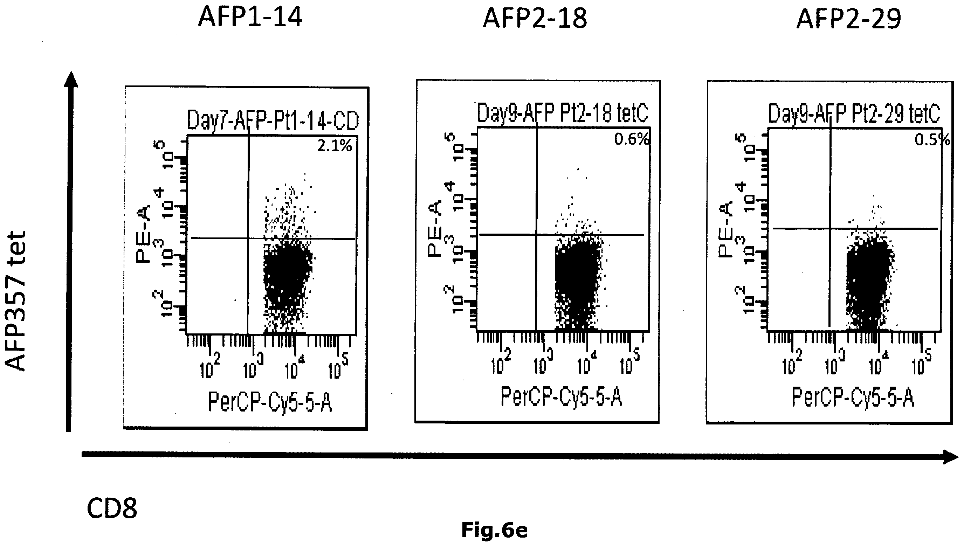

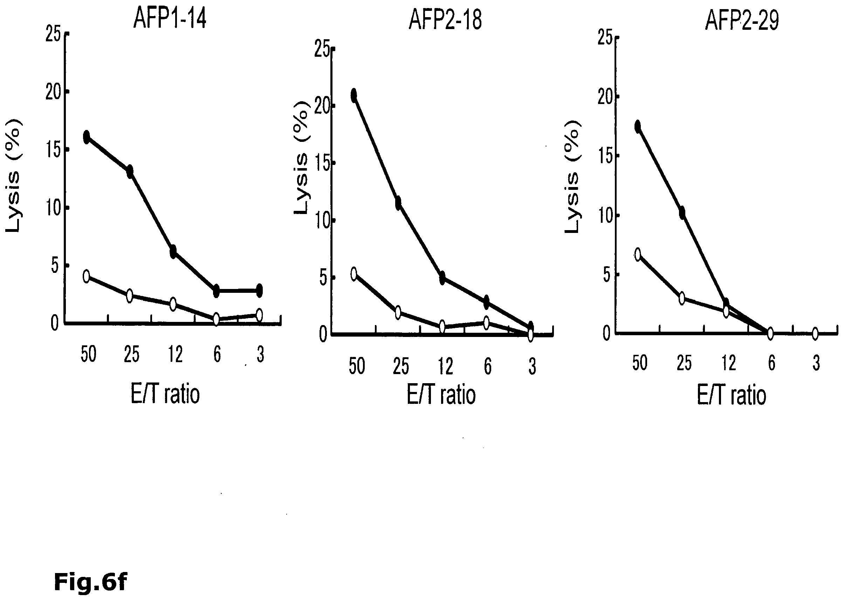

[Fig. 6] Analysis of peptide vaccine-specific TCRs from AFP peptide-vaccinated HCC patients by hTEC10 system. (a) Clinical response of patient 1. Patient 1 was vaccinated with AFP₃₅₇ and AFP₄₀₃ peptides biweekly for 72 weeks. The clinical response was monitored by serum AFP levels before and after the AFP-derived peptide vaccination. The serum AFP level was measured by enzyme immunoassay (upper). The clinical response of patient 1 was also monitored by MRI (lower). Blue arrows show the date when the representative MRI examinations were performed. Red arrowheads show the lesion of HCC in liver. (b) Clinical response of patient 2. Patient 2 was vaccinated with AFP₃₅₇ and AFP₄₀₃ peptides biweekly for 88 weeks. The clinical response of the patient was monitored by serum AFP level as determined by enzyme immunoassay (upper). The clinical response of patient 2 was also monitored by CT (lower). The arrows in the upper part showing the results of monitoring of the AFP level over time show the date when the representative CT examinations were performed. The arrows in the CT image (lower) show the metastatic lesions of HCC in the abdominal wall and lung. (c) Detection of AFP peptide-specific CD8⁺ T cells of PBL in HCC patients. PBL from HCC patients who had been treated with the peptide vaccine were stimulated with AFP₃₅₇ peptide for three weeks. After stimulation, the cells were stained with an anti-CD8 antibody and HLA-A*2402/AFP₃₅₇ peptide tetramers and analyzed using FACS. (d) Repertoire of HLA-A*2402/AFP₃₅₇ peptide tetramer⁺ CD8⁺ T cells. The repertoire was analyzed using the IMGT/V-Quest tool (http://www.imgt.org/). "n" is the number of analyzed T cell clones. "r" represents the repertoire number. An asterisk denotes that the repertoire was obtained from only a single T cell clone. (e) Expression of AFP₃₅₇-specific TCRs on TCR-transduced primary T cells. Primary T cells from healthy donors were retrovirally transduced with three kinds of AFP-specific TCR cDNAs (AFP1-14, AFP2-18, and AFP2-29), and TCR expression was analyzed by staining with an anti-CD8 antibody and HLA-A*2402/AFP₃₅₇ peptide tetramers. (f) Cytotoxicity of TCR-transduced primary T cells. AFP₃₅₇ peptide-specific TCR transduced T cells were incubated with ⁵¹Cr-labeled C1RA24 cells that had been pulsed with or without AFP₃₅₇ peptide. The closed and open circles show the cytotoxicity against C1R-A24 cells pulsed with and without AFP₃₅₇ peptide, respectively. ⁵¹Cr release was measured 4 hours later, and lysis (%) was calculated as described in the Methods section.

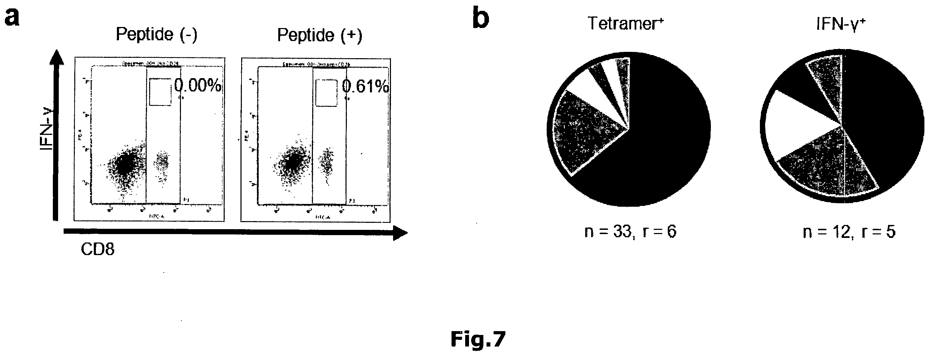

[Fig. 7] Repertoire analysis of cytokine secreting CD8⁺ T cells by stimulation with a specific peptide. (a) PBLs from donor F were stimulated with the BRLF-1 peptide for 14 days. After *in vitro* stimulation, the PBLs were re-stimulated with an anti-CD28 antibody with or without the BRLF-1 peptide for 6 hours. The secretion of IFN-γ by the responding cells was analyzed with an IFN-γ secretion assay kit. (b) The repertoires of IFN-γ⁺ CD8⁺ T cells were analyzed and compared with those from the BRLF-1-specific MHC/p tetramer⁺ CD8⁺ T cells of donor F. "n" is the number of analyzed T cell clones. "r" represents the repertoire number. The same color denotes the same Vα/Vβ repertoires.

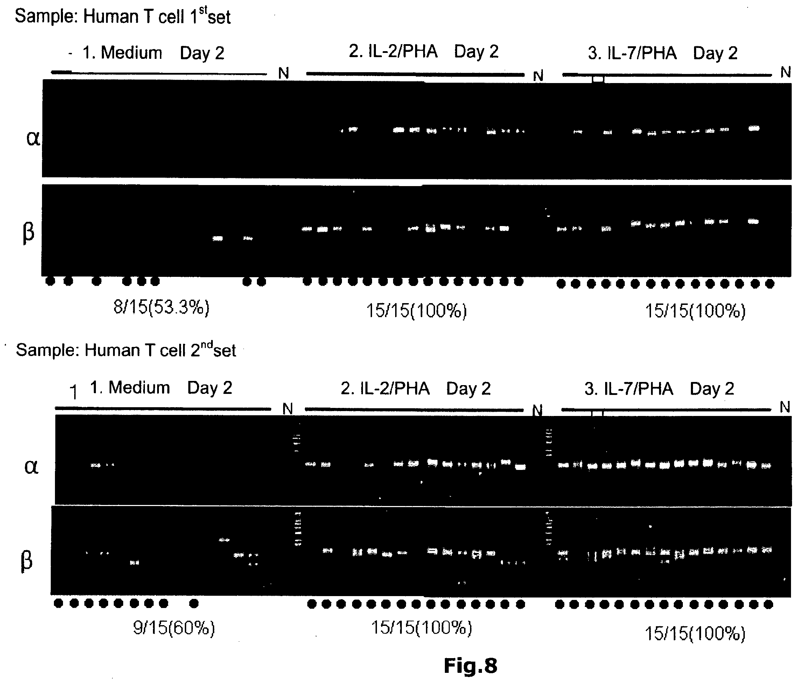

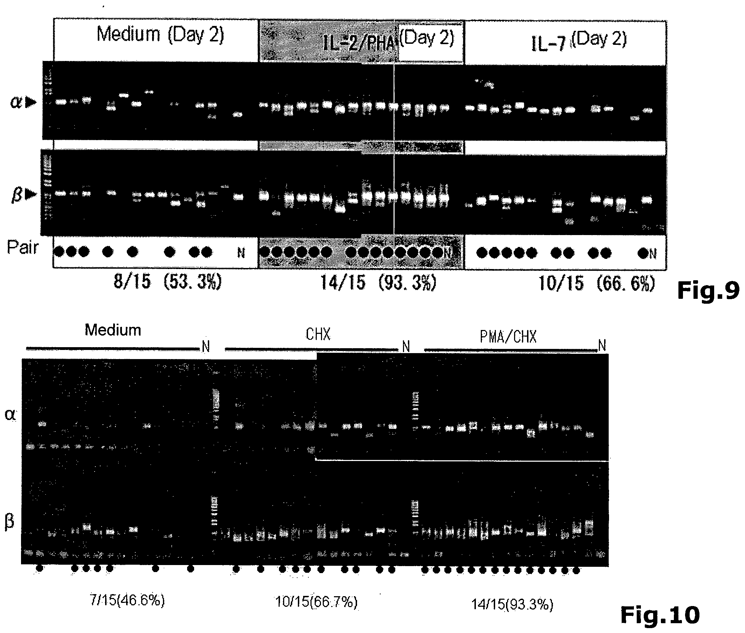

[Fig. 8] Amplification ratios obtained with IL-2/PHA and IL-7/PHA. PBLs as a population were cultured for two days in the presence of the stimulant, and then CD3-positive T cells were each sorted into a tube as a single cell, and subjected to RT-PCR. A sample of 10 µl obtained from each tube was subjected to electrophoresis using 1% agarose gel, and stained with ethidium bromide (EtBr). The closed circles indicate the systems where the TCR α/β cDNA pair could be amplified, and the letters N indicate systems not containing the cell (negative control) (the same shall apply to Figs. 9 and 10). The pair amplification ratios were 30/30 (100%) when the cells were stimulated with Il-2/PHA, and 30/30 (100%) when the cells were stimulated with IL-7/PHA. When the cells were cultured only with the medium (no stimulation) for two days, the amplification ratio was 17/30 (56.7%).
[Fig. 9] Amplification ratios obtained with IL-7 alone. The experiment was carried out in the same manner as that used for the experiment of which results are shown in Fig. 8, except that the stimulant was changed. The pair amplification ratios obtained after culture for 2 days were 53.3% when the cells were cultured only with the medium (no stimulation), 93.3% when the cells were stimulated with Il-2 and PHA, and 66.6% when the cells were stimulated with IL-7 alone.
[Fig. 10] Amplification ratios obtained with CHX and PHA/CHX. The experiment was carried out in the same manner as that used for the experiment of which results are shown in Fig. 8, except that human CD3⁺ cells were used, and the culture time in the presence of the stimulant was 12 hours. The pair amplification ratios obtained after culture for 12 hours were 46.6% when the cells were cultured only with the medium (no stimulation), 66.7% when the cells were stimulated with CHX alone, and 93.3% when the cells were stimulated with PMA/CHX.

### Modes for Carrying out the Invention

In the present invention and the present specification, the ranges indicated with "to" include the values indicated on both sides of "to", unless especially indicated.

The present invention provides a method for cloning a TCR gene. More specifically, the present invention provides a method for producing a TCR gene specific to an antigen A as defined in claim 1.

The step 1) is a step of stimulating a group of T cells or one T cell under a condition effective for amplification of a TCR gene. The condition effective for amplification of a TCR gene is a condition for treating the T cells or cell so that PCR (polymerase chain reaction) can be effectively performed in the step 3), and this is usually a condition for increasing mRNA that can serve as a template to an amount sufficient for performing PCR.

This condition typically consists of maintaining the T cells or cell in the presence of at least IL-2, preferably in the presence of IL-2 and PHA, for a period effective for the treatment. If the T cells are maintained as a population, mRNAs that can serve as a template may be increased to an amount sufficient for performing PCR.

IL-2 is generally known to have actions of activating monocytes and macrophages, promoting proliferation and antibody production ability of B cells, and proliferating and activating T cells. PHA (phytohemagglutinin) is generally known as a mitogen. A substance having a similar activity can also be used as a substitute for these.

According to the investigation of the inventors of the present invention, the condition effective for amplification of a TCR gene in the step 1) consists of at least maintaining a group of cells or cell in the presence of
interleukin 2(IL-2) and phytohemagglutinin (PHA), or interleukin 7 (IL-7) and phytohemagglutinin (PHA); or
phorbol 12-myristate 13-acetate (PMA) and cycloheximide (CHX).

The stimulants are used as a combination of them for increasing the amplification ratio of the antigen-specific TCR α/β cDNA pair in the step 3). According to the investigation of the inventors of the present invention, the combinations with which high amplification ratio of the antigen-specific TCR α/β cDNA pair was confirmed are the combination of IL-2 or IL-7 and PHA, and the combination of PMA and CHX.

The stimulant can be used by dissolving it in a medium (for example, RPMI1640) or buffer that enables culture and maintenance of T cells at an appropriate concentration. When IL-2 or IL-7 is used, the concentration thereof may be, for example, 10 to 5000 IU/ml, more specifically 30 to 1000 IU/ml, still more specifically 50 to 500 IU/ml. When PHA is used, the concentration thereof may be, for example, 0.1 to 100 µg/ml, more specifically 0.5 to 50 µg/ml, still more specifically 1 to 9 µg/ml. When PMA is used, the concentration thereof may be, for example, 0.1 to 100 ng/ml, more specifically 0.5 to 50 ng/ml, still more specifically 1 to 20 ng/ml. When CHX is used, the concentration thereof may be, for example, 0.1 to 100 µg/ml, more specifically 0.5 to 50 µg/ml, still more specifically 1 ng/ml to 20 µg/ml. The medium or buffer may further contain blood serum (for example, heat-inactivated fetal bovine serum) and antibiotics (for example, streptomycin and penicillin).

According to the investigation of the inventors of the present invention, when IL-2 and PHA are used under the conditions described in the examples, the period effective for the treatment is 0.5 to 3 days, preferably 24 to 64 hours, more preferably 36 to 60 hours, typically 2 days.

The other conditions such as temperature and 5% CO₂ atmosphere may be as usual conditions for culturing T cells.

According to one embodiment of the present invention, T cells as a population, namely, a group of T cells, are cultured or stimulated in the step 1). When a group of T cells are stimulated, the efficiency of the amplification performed in the step 3) can be improved by the influence of an interaction of neighboring cells (cell adhesion, humoral factors such as cytokines). When T cells are stimulated as a population, they can be stimulated by suspending them in a medium containing the stimulant at a usual cell density suitable for the culture, for example, 1 x 10⁵ to 1 x 10⁷ cells/ml, preferably 5 x 10⁵ to 5 x 10⁶ cells/ml, inoculating the suspension in an appropriate culture vessel in an appropriate volume, and culturing the cells.

As the culture vessel used for the culture mentioned above, for example, a commercially available 24-well plate can be used, and by inoculating 1 ml of the cell suspension containing 1 x 10⁶ cells/ml to each well, a population of the T cells of 1 x 10⁶ cells/well can be stimulated. A commercially available 96-well plate can also be used, and in this case, by inoculating 200 µl of the cell suspension containing 1 x 10⁶ cells/ml to each well, a population of the T cells of 2 x 10⁴ cells/well can be stimulated.

According to another embodiment of the present invention, single T cells are stimulated in the step 1). In this case, the method of the present invention can be performed by carrying out the step 2) first to identify T cells and separate them into appropriate vessels as single cells, and then carrying out the step 1). Since it is obvious that even when the T cells are stimulated as single cells, they can be stimulated with the stimulant, it is expected that the amplification ratio of the antigen-specific TCR α/β cDNA pair will be increased in the step 3) compared with the case where the cells were not stimulated (according to the investigation of the inventors of the present invention, the amplification ratio of the antigen-specific TCR α/β cDNA pair was 36.4% in an experiment where the cells were cultured for 12 hours as a population without stimulation (using only a medium)). Further, there have been reported a method of stimulating cells with an artificial cell that is made to secret a cytokine and express a costimulatory molecule (CD80, CD137L) in a tube into with the cells are sorted (Proc. Natl. Acad. Sci. USA, 2012 Mar 6; 109 (10):3885-90), and so forth, and if such a method is applied, the effect may be obtained at a high degree even when the cells are stimulated after they are separated into single cells as high as that obtainable by stimulating the cells as a group of T cells. When a term "single" or "one" is used in the present invention concerning state of cell, it means that the cell is in an environment not containing any other cells, unless especially stated. Further, when the term "population" or "group" is used, the cells are in an environment where a plurality of cells exist.

The step 2) is a step of identifying an objective T cell, and sorting it into a vessel for PCR as a single cell. This step can be carried out by various existing means, for example, flow cytometry or immunospot array assay on a chip (ISAAC) method.

Flow cytometry is a technique of dispersing microparticles in a fluid, thinly flowing the fluid, and optically analyzing each particle, and it also enables selective collection of the microparticles. It is also commonly used for analysis of cell surface markers, and it is typically used for classifying cells positive for two kinds of surface markers.

According to one disclosure, the step 2) is performed by using a multimer (typically tetramer) of a complex of a major histocompatibility complex (MHC) molecule and an antigen A-derived antigen peptide (p) (MHC/p tetramer) and anti-CD4 antibody or anti-CD8 antibody, and sorting T cells reactive to the both by flow cytometry. In the present invention and the present specification, the multimer of MHC molecule and p may be explained by exemplifying the MHC/p tetramer, but the explanation for it may also be applied to other multimers such as pentamer, unless especially indicated.

In the examples mentioned in this specification, results of quick and direct cloning of an antigen-specific TCR using an MHC/p tetramer are shown. This embodiment is advantageous in that specific T cells can be definitely sorted. Most of the currently available MHC/p tetramers are a tetramer comprising peptides bound to an MHC class I molecule. Such a tetramer binds to a CD8-positive T cell. Therefore, in such a case, T cells reactive to the MHC/p tetramer and CD8-positive are preferably sorted. Further, antigen-specific T cells can also be detected by using a tetramer comprising an MHC class II molecule bound with peptides. This MHC/p tetramer binds to a CD4-positive T cell. Therefore, when this tetramer as a complex of the MHC class II molecule and peptides is used, CD4-positive antigen-specific T cells are preferably sorted.

According to another disclosure, the step 2) is carried out by sorting CD8⁺ T cells that react with a peptide to secrete IFN-γ by flow cytometry using an anti-interferon γ (IFN-γ) antibody, and anti-CD4 antibody or anti-CD8 antibody. Although availability of MHC/p tetramer is limited, the method of the present invention may be used for detecting an antigen-specific T cell without using any MHC/p tetramer. In this embodiment, it is preferable to stimulate PBLs obtained from a donor with a stimulant, and then stimulate again the stimulated PBLs in the presence of an antigen and anti-CD28 antibody. This enables staining of an IFN-γ-secreting cell with an IFN-γ secretion assay kit, and a CD4-positive or CD8-positive, and IFN-γ-positive T cell can be identified and sorted as an objective T cell.

The step 2) is carried out by the ISAAC method. This method is performed by using a microwell array having a plurality of wells of such a size that only one T cell is contained in each well on one main surface of a substrate. As for the method of using a microwell array, the previous patent application of the inventors of the present invention can be referred to. In this method, as a substance showing binding property (binding substance) for at least a part of a substance that is produced when a T cell has recognized an antigen (producing substance), an antibody directed to the producing substance can be used. Detection of the presence or absence of the producing substance is performed by using a substance that specifically binds to the producing substance, or a substance that specifically binds to the binding substance. A typical example of the substance for the detection is an antibody. Specifically, one T cell is placed in each well on a microwell array, and the T cell is stimulated with a peptide on the chip. The surface of the chip has been coated with an anti-IFN-γ antibody beforehand. The stimulated T cell secrets IFN-γ, and the secreted IFN-γ is trapped by the anti-IFN-γ antibody on the surface of the chip around the well. The trapped IFN-γ is detected by binding a fluorescence-labeled anti-IFN-γ antibody. The IFN-γ-secreting T cell identified as described above is collected with a capillary, and used for the amplification of TCR.

The step 3) is a step of subjecting the one activated (synonymous with stimulated) antigen A-specific T cell in a vessel to PCR to amplify a gene of TCR specific to the antigen A. The identified cell may be proliferated and then subjected to PCR, but various techniques of PCR enabling amplification of cDNA from one or several cells are known, and such known techniques can also be used in the present invention. Typically, the cell is lysed first, and then cDNA is synthesized from mRNA by reverse transcription reaction using dT adapter primers (RT dT Primer 2). The obtained cDNA is used after amplification or as it is as a template to perform real-time PCR (quantitative PCR, qPCR) using appropriately designed primers.

According to the present invention, the steps 1), 2), and 3) are carried out in this order.

The method of the present invention may further comprises a step of introducing the obtained TCR gene into another T cell not expressing TCR, and verifying antigen specificity of the expressed TCR.

The method of the present invention can also be performed as a method for producing a recombinant T cell specific to an antigen A, which comprises, besides the steps of the aforementioned production method, a step of introducing the obtained gene of TCR specific to an antigen A into another T cell to obtain a recombinant T cell specific to the antigen A.

The T cell as the object of the recombination may be derived from a subject (patient) with a disease or condition treatable with a TCR gene therapy. Typical examples of such a disease or condition are cancers and infectious diseases, and cancers are preferred. The antigen used for the present invention may be a cancer-associated antigen. Examples of the cancer-associated antigen include, besides those mentioned in the examples of this specification, WT1, CEA, CA 19-9, CA125, PSA, CA72-4, SCC, MK-1, MUC-1, p53, HER2, G250, gp-100, MAGE, BAGE, SART, MART, MYCN, BCR-ABL, TRP, LAGE, GAGE, and NY-ESO1.

Diseases for which application of the present invention can be expected include cancers and infectious diseases, and the cancers include adult cancers and infant cancers, including gastrointestinal carcinoma, lung cancer intractable esophageal carcinoma, head and neck cancer, ovarian cancer, multiple myeloma, and so forth. The infectious diseases include viral infectious diseases (for example, acquired immunodeficiency syndrome (AIDS), adult T cell leukemia, Ebola hemorrhagic fever, influenza, viral hepatitis, viral meningitis, yellow fever, cold syndrome, rabies, cytomegalovirus infection, severe acute respiratory syndrome (SARS), progressive multifocal leucoencephalopathy, varicella, herpes zoster, hand-foot-and-mouth disease, dengue fever, erythema infectiosum, infectious mononucleosis, variola, rubella, acute anterior poliomyelitis (polio), measles, pharyngo-conjunctival fever (swimming pool sickness), Marburg hemorrhagic fever, hantavirus hemorrhagic fever with renal syndrome, Lassa fever, mumps, West Nile fever, herpangina, chikungunya hemorrhagic fever), bacterial infectious diseases, rickettsial infectious diseases, parasitic infectious diseases, and prion diseases.

The term "treatment" used for the method of the present invention concerning disease or condition includes reduction of risk of development, prophylaxis, treatment, and suppression of advance, unless especially indicated.

Hereafter, the present invention will be explained with reference to examples.

### Example 1

### [Methods]

### Healthy donor and HLA typing

Human experiments were performed with the approval of the Ethical Committee at the University of Toyama. Informed consent was obtained from all subjects. Peripheral blood lymphocytes (PBLs) were isolated from heparinized blood samples by density gradient centrifugation using Ficoll-Hypaque (Immuno-Biological Laboratories). Screening for HLA-A24 haplotype positivity was performed by staining PBLs with an anti-HLA-A24 antibody (One Lambda) followed by a FITC-conjugated anti-mouse IgG antibody (ICN/Cappel) and analysis via flow cytometry.

### Patients with peptide vaccination

The clinical trial (trial registration: UMIN000003514) using HLA-A24 restricted AFP₃₅₇ (EYSRRHPQL, SEQ ID NO: 1) and AFP₄₀₃ (KYIQESQAL, SEQ ID NO: 2) peptide vaccines was performed at Kanazawa University Hospital. Patients with verified radiological diagnoses of HCC stage III or IV were enrolled in this study. The patients each received 3.0 mg of AFP-derived peptide vaccine per dose. The peptides, which were synthesized as GMP grade at Neo MPS, Inc. (San Diego, California), were administered as an emulsified solution containing incomplete Freund's adjuvant (Montanide ISA-51 VG; SEPPIC, Paris, France) by biweekly subcutaneous immunization. The clinical responses were monitored by serum AFP value, dynamic CT or MRI and evaluated according to the Response Evaluation Criteria in Solid Tumors, version 1.1. All patients provided written informed consent to participate in the study in accordance with the Helsinki Declaration, and this study was approved by the regional ethics committee (Medical Ethics Committee of Kanazawa University, No. 858).

Blood samples from the patients were tested for HBsAg and HCVAb using commercial immunoassays (Fuji Rebio). HLA-based typing of PBLs from patients was performed using the polymerase chain reaction-reverse sequence-specific oligonucleotide (PCR-RSSO) method. The serum AFP level was measured by enzyme immunoassay (Abbott Japan). The PBLs from patients were isolated as described previously (Reference 14), resuspended in RPMI 1640 medium containing 90% FCS and 10% dimethyl sulfoxide and cryopreserved until use.

### Cell lines

RPMI 1640 and DMEM medium (Wako Pure Chemical) were supplemented with 10% heat-inactivated fetal bovine serum (Biowest), 100 µg/ml streptomycin, and 100 U/ml penicillin, and used for culture of the cells. Human CD8 (hCD8)-expressing TG40 cells (kindly provided by Dr. Ueno, Kumamoto University; with permission from Dr. Saito, Riken) and T2-A24 cells (kindly provided by Dr. Kuzushima, Aichi Cancer Center Laboratory) were maintained in RPMI 1640 medium. PLAT-E (kindly provided by Dr. Kitamura, University of Tokyo) and Phoenix-A (kindly provided by Dr. G. Nolan, Stanford University) were maintained in DMEM medium.

### Stimulation of cells

### Stimulation with IL-2/PHA

A cell suspension of 1 x 10⁶ cells/ml was inoculated in a volume of 1 ml to each well of a 24-well plate (1 x 10⁶ cells/well), and the cells were stimulated. The stimulation was performed by culturing the cells in the RPMI 1640 medium (Wako Pure Chemical) supplemented with 10% heat-inactivated fetal bovine serum (Biowest), 100 µg/ml streptomycin, 100 U/ml penicillin, 100 IU/ml recombinant human IL-2 (Peprotech, Cat. 200-02) and 3 µg/ml PHA (Wako Pure Chemical) at 37°C under 5% CO₂ for two days.

### Stimulation with CHX/PMA

A cell suspension of 1 x 10⁶ cells/ml was inoculated in a volume of 200 µl to each well of a 96-well plate (Flat bottom) (2 x 10⁴ cells/well), and the cells were stimulated. The stimulation was performed by culturing the cells in the RPMI 1640 medium (Wako Pure Chemical) supplemented with 10% heat-inactivated fetal bovine serum (Biowest), 100 µg/ml streptomycin, 100 U/ml penicillin, 10 µg/ml CHX (Wako Pure Chemical) and 10 ng/ml PHA (Wako Pure Chemical) at 37°C under 5% CO₂ for a predetermined time. After the stimulation, the cells were subjected to CD3⁺ positive selection using an automatic magnetic cell separator (autoMACS, Miltenyi Biotec), and then subjected to TCR amplification.

### Antibody and MHC/p tetramer staining

EBV-specific T cells were stained with PE-conjugated HLA-A24/peptide tetramers. The sequences of the EBV peptides used were as follows: TYPVLEEMF (BRLF-1 198-206, SEQ ID NO: 3), DYNFVKQLF (BMLF-1 320-328, SEQ ID NO: 4), IYVLVMLVL (LMP2 222-230, SEQ ID NO: 5), RYSIFFCYM (EBNA3A 246-254, SEQ ID NO: 6), and TYSAGIVQI (EBNA3B 217-225, SEQ ID NO: 7). AFP-specific T cells were stained with the PE-conjugated HLA-A24/peptide (AFP 357-365) tetramer (Reference 13). All MHC/p tetramers were purchased from MBL. A FITC-conjugated anti-CD8 antibody (MBL), an anti-CD3ε antibody (eBioscience), APC-conjugated streptavidin (eBioscience), and a PE-conjugated anti-CD69 antibody (eBioscience) were used for flow cytometry.

### Single cell RT-PCR

PBLs, which had been stimulated with IL-2 and PHA for 2 days, were stained with the MHC/p tetramer, and MHC/p tetramer-positive cells were each sorted as single cells each into a MicroAmp® reaction tube (Applied Biosystems) that contained a cell lysis solution composed of 29.2 µg Dynabeads Oligo(dT) (Reference 25) (Invitrogen), 2.9 µl Lysis/Binding Buffer (Invitrogen) and 0.29 pmol each gene specific primer using FACSAriaII (Becton Dickinson).

The sequences of the primers were as follows:
alpha-RT (5'-AGCAGTGTTTGGCAGCTCTT-3', SEQ ID NO: 8),
beta1-RT (5'-CTGGCAAAAGAAGAATGTGT-3', SEQ ID NO: 9), and
beta2-RT (5'-ACACAGATTGGGAGCAGGTA-3', SEQ ID NO: 10).

The cells were lysed in a tube. Poly-A RNA was bound to Oligo(dT) on the Dynabeads. The Dynabeads were then transferred into a solution containing 4.0 U SuperScriptIII (Invitrogen), 0.3 U murine RNase inhibitor (New England Biolabs), 0.5 mM each dNTP, 5 mM DTT, 0.2% Triton X-100, and 1×First-Strand Buffer (Invitrogen). The reverse transcription (RT) reaction was performed for 40 min at 50°C. After the RT reaction, the Dynabeads were transferred into another solution containing 8 U terminal deoxynucleotidyl transferase (Roche), 0.5 mM dGTP, 0.4 U murine RNase inhibitor, 4 mM MgCl₂, 0.2% Triton-X 100, and 5% P-K buffer [1 M K₂HPO₄ and 1 M KH₂PO₄, pH 7.0], and incubated for 40 min at 37°C to add a poly-dG tail at the 3'-end of the cDNA. The Dynabeads were then transferred into a new PCR tube containing the first PCR reaction mix. The first PCR was performed using PrimeSTAR HS DNA polymerase (TaKaRa) according to the manufacturer's instructions with AP-1, alpha-1st, betal-1st and beta2-1st primers.

The PCR cycles for AP-1 (5'-ACAGCAGGTCAGTCAAGCAGTAGCAGCAGTTCGATAACTTCGAATTCTGCAGTCGACGG TACCGCGGGCCCGGGATCCCCCCCCCCCCCDN-3', SEQ ID NO: 11), alpha-lst (5'-AGAGGGAGAAGAGGGGCAAT-3', SEQ ID NO: 12), beta1-1st (5'-CCATGACGGGTTAGAAGCTC-3', SEQ ID NO: 13), and beta2-1st (5'-GGATGAAGAATGACCTGGGAT-3', SEQ ID NO: 14) were as follows: 5 min at 95°C, followed by 30 cycles of 15 sec at 95°C, 5 sec at 60°C, and 1 min 30 sec at 72°C.

The resultant PCR mixtures were diluted 100-fold with water, and 2 µl of the diluted PCR mixtures were added to 23 µl of the nested PCR mix as template DNA. The nested PCR was performed in a similar reaction mix to that of the first PCR but with the adapter primer AP-2 (5'-AGCAGTAGCAGCAGTTCGATAA-3', SEQ ID NO: 15) and a specific primer for the constant region of TCR alpha (alpha-Nest; 5'-GGTGAATAGGCAGACAGACTT-3', SEQ ID NO: 16) or specific primer for the constant region of TCR beta (beta-Nest; 5'-GTGGCCAGGCACACCAGTGT-3', SEQ ID NO: 17). The PCR cycles were as follows: 1 min at 98°C, followed by 35 cycles of 15 sec at 98°C, 5 sec at 60°C, and 45 sec at 72°C.

The PCR products were then analyzed using the alpha-nest or beta-nest primer by either direct sequencing or sequencing after subcloning into an expression vector. The TCR repertoire was analyzed using the IMGT/V-Quest tool (http://www.imgt.org/) (Reference 15).

### Retroviral transfection

cDNAs encoding the TCR α or β chain were independently inserted into a pMX vector (kindly provided by Dr. Kitamura, University of Tokyo) and then transfected into a retroviral packaging cell line, PLAT-E, with FuGENE 6 (Roche). The culture supernatant containing recombinant retroviruses from the transfected PLAT-E cells was collected 72 hours after transfection, and added to hCD8-TG40 cells together with polybrene (Sigma-Aldrich). Whether TCR was expressed in the hCD8-TG40 cells infected with the recombinant retroviruses was monitored by the cell surface expression of CD3ε and EBV-specific MHC/p tetramer binding to the cells as analyzed by flow cytometry. For transduction to human PBLs, the TCR α and TCR β chains were linked via a viral F2A sequence (Reference 16), cloned into a pMX-IRES-EGFP vector (kindly provided by Dr. Kitamura, University of Tokyo) and transfected into the Phoenix A retroviral packaging cell line.

### Determination of antigen-specificity of cloned TCRs

The antigen-specificity of the cloned TCR α/β pairs was analyzed using the CD69 induction assay and/or MHC/p tetramer staining. Briefly, TCR-expressing hCD8-TG40 cells were incubated overnight with HLA-A24⁺ PBLs in the presence of each of the EBV peptides (BRLF-1, BMLF-1, LMP2, EBNA3A or EBNA3B) at 37°C in 5% CO₂. After incubation, the cell surface expression of CD69 was analyzed by flow cytometry. In case of antigen-specificity for LMP2, the cells were stained with the fluorescence-labeled LMP2-specific HLA-A24 tetramer, and this binding was analyzed by using a flow cytometer.

### Preparation of PBLs transduced with cloned TCRs

5 x 10⁵ PBLs were stimulated *in vitro* with CD3CD28 Dynabeads (Invitrogen) and 30 U/ml recombinant hIL-2 (Peprotech) according to the manufacturer's instructions. Two days later, TCR-encoding retroviral supernatant was added to plates that had been coated overnight with 50 µg/ml retronectin (TaKaRa). The supernatant containing the retroviruses was spin-loaded onto the plate by centrifuging for 2 hours at 1900 x g at 32°C. The stimulated PBLs were washed, and 0.5 x 10⁶/ml of these cells were added to each well in the retrovirus-loaded plates. The plates were spun at 1000 x g at 32°C for 10 minutes and incubated overnight at 37°C in 5% CO₂. The next day (day 3), the PBLs were transferred onto newly prepared retroviral-coated plates as on day 2 and incubated at 37°C in 5% CO₂. On day 10, the TCR-transduced PBLs were evaluated for expression of the appropriate TCR by MHC/p tetramer staining and flow cytometry.

### CTL assay

The cytotoxicity of the TCR-transduced PBLs was measured using the calcein-AM (Wako Pure Chemical Industries) release assay. Briefly, peptide-loaded T2-A24 target cells were labeled with calcein-AM for 30 min at 37°C. Then, the target cells and TCR-transduced PBLs (effector cells) were plated in 96-well plates at the indicated effector-to-target (E/T) ratios and incubated for 4 hours at 37°C in humidified air containing 5% CO₂. After incubation, the supernatants were transferred to new wells, and fluorescence was measured using a FLUOstar OPTIMA microplate reader (BMG LABTECH). The percentage of cytotoxicity was calculated using the following formula: % lysis = (F experiment - F spontaneous) / (F maximal - F spontaneous) × 100. Assays were performed in triplicate. In the case of the AFP-specific TCR, the cytotoxicity of the TCR-transduced PBLs was measured by the ⁵¹Cr release assay (Reference 13).

### [Results]

### Rapid cloning and functional evaluation of antigen-specific TCRs from a single antigen-specific human CD8⁺ T cell

Fig. 1a shows the schematic of the rapid cloning and functional assay system established by the inventors of the present invention, which can obtain TCR α/β cDNA pairs from a single antigen-specific human T cell and confirm their antigen specificity within 10 days. The inventors of the present invention designated this system as the hTEC10 system (human TCR efficient cloning within 10 days). In this system, human antigen-specific T cells are detected by staining with antigen-specific MHC/p tetramers or analysis of cytokine secretion (Fig. 1a, left), and single cells are obtained by FACS. TCR cDNA is amplified from single cells (Fig. 1a, center), cloned into an expression vector, and transduced into the TCR-negative T cell line TG40. The antigen specificity of the TCR is then assessed by staining the transduced TG40s with MHC/p tetramers (Fig. 1a, right) and analyzing CD69 expression. This entire process can be performed within 10 days. As shown in Fig. 1b, amplification of TCR cDNA from single cells is extremely efficient.

To evaluate the hTEC10 system for analyzing T cells in human diseases, the inventors of the present invention first analyzed the EBV-specific CD8⁺ T cells derived from HLA-A24⁺ latent healthy donors. To date, five HLA-A*2402-restricted EBV epitopes, BRLF-1, BMLF-1, LMP2, EBNA3A and EBNA3B, have been identified (Reference 9). Thus, the inventors of the present invention used a HLA-A*2402 restricted tetramer mix of the five EB virus epitopes to detect EBV-specific CD8⁺ T cells in the present study. The inventors of the present invention detected various frequencies (0.64% to 0.00%) of MHC/p tetramer-positive cells within the CD8⁺ T cell populations from 19 HLA-A24-positive donors (Fig. 2a and Table 1).

**[Table 1]**

| Frequency of EBV-specific CD8⁺ T cells | | | | | |
|---|---|---|---|---|---|
| Donor | HLA-A24^{a} | Tet⁺%^{b} | Donor | HLA-A24 | Tet⁺% |
| A | + | 0.03% | N | + | 0.01% |
| B | + | 0.66% | O | - | ND |
| C | - | 0.01% | P | + | 0.02% |
| D | + | 0.05% | Q | + | 0.08% |
| E | + | 0.26% | R | + | 0.03% |
| F | + | 0.17% | S | - | ND |
| G | - | ND^{c} | T | + | 0.08% |
| H | - | ND | U | + | 0.56% |
| I | + | 0.07% | V | - | ND |
| J | + | 0.37% | W | + | 0.34% |
| K | + | 0.17% | X | + | 0.00% |
| L | + | 0.05% | Y | + | 0.05% |
| M | + | 0.01% | Z | - | ND |

| | | | | | |
|---|---|---|---|---|---|
| ^{a}HLA-A24 haplotype positivity was determined by staining PBL from healthy donors with an anti-HLA-A24 antibody. ^{b}Percentages of tetramer-positive cells in CD8⁺ T cells of healthy donor PBL were determined by staining with the HLA-A*2402 EBV tetramer mixture. ^{c}ND, not determined. | | | | | |

The inventors of the present invention then used FACS to single cell sort the MHC/p tetramer-positive cells from PBLs of 10 donors whose frequencies of EBV-specific MHC/p tetramer-positive cells were more than 0.06% of the CD8⁺ T cell population. The inventors of the present invention amplified 444 pairs of TCR α and β cDNAs from sorted single cells using the 5'-RACE method (Non-patent document 7 mentioned above). The inventors of the present invention then analyzed the sequences of EBV-specific TCR pairs from each donor. The inventors of the present invention found that the diversity of the EBV-specific TCRs was highly restricted (1 to 10 in each donor) (Fig. 2b, 3). Because the TCR repertoire of MHC/p tetramer⁻ cells was not skewed toward any Vα/Vβ subgroup (Fig. 3), the skewing of the TCR repertoire in MHC/p tetramer⁺ cells was not due to PCR bias by the 5'-RACE method.

It is important to note that the system of the inventors of the present invention can clone the rare antigen-specific T cell clones (indicated by asterisks in Fig. 2b) that may be missed with conventional cloning methods. It is also of note that the number of T cell clones obtained from each donor was inversely correlated with the percentage of MHC/p tetramer⁺ CD8⁺ T cells (Fig. 2c), suggesting that specific clones were expanded from each donor to regulate EBV latency.

### Determination of the antigen specificity of the cloned TCRs

To determine the antigen specificity of the cloned TCRs, the inventors of the present invention first transferred the cDNAs into TG40 cells and stained them using the MHC/p tetramer mixture. Ninety-five percent of TCRs that were expressed on TG40s bound to the MHC/p tetramer mixture (Fig. 2d). The inventors of the present invention then determined the antigenic peptide specificity of the cloned TCRs using the CD69 induction assay. The inventors of the present invention incubated TCR-expressing TG40 cells with HLA-A24⁺ peripheral blood lymphocyte (PBL) in the presence of each EBV peptide and examined the expression of CD69, an early lymphocyte activation marker. As shown in Fig. 2e, for TCR E-21, CD69 expression was significantly increased with the EBNA3A peptide but not with other peptides. In the case of TCR F-7, CD69 expression was significantly increased with the BRLF-1 peptide but not with other peptides. These results show that the E-21 TCR is specific for EBNA3A and that the F-7 TCR is specific for BRLF-1. When the inventors of the present invention analyzed the cognate antigens of 379 TCRs using the CD69 induction assay, they found that the percentages of BRLF-1, BMLF-1, EBNA3A, EBNA3B and LMP-2-specific TCRs among EBV-specific TCRs were 57.1, 21.1, 18.8, 2.0, and 1.1%, respectively (Table 2). These data are in agreement with previous reports (References 10 and 11).

**[Table 2]**

| Antigenic determinant of the obtained EBV-specific TCRs. | | | | | | |
|---|---|---|---|---|---|---|
| Antigen | | | | | | |
| Donor | BRLF-1^{a} | BMLF-1 ^{a} | LMP-2 ^{a} | EBNA3A ^{a} | EBNA3B ^{a} | Total ^{b} |
| B | 89^{c} | | | | | 89 |
| E | 1 | 1 | | 40 | | 42 |
| F | 33 | | | | | 33 |
| I | 12 | 1 | 1 | | 1 | 15 |
| J | 16 | 3 | | | | 19 |
| K | | 24 | 1 | | | 25 |
| Q | 20 | 12 | | 1 | 5 | 38 |
| T | 5 | 7 | | | | 12 |
| U | 68 | | | | | 68 |
| W | 4 | | | 34 | | 38 |
| Total | 289 | 48 | 2 | 75 | 6 | 379 |

| | | | | | | |
|---|---|---|---|---|---|---|
| ^{a}EB virus-derived T cell antigen determinants. ^{b}Total number of obtained TCRs from sorted cells that were stained with the HLA-A*2402 EBV tetramer mixture. ^{c}Number of antigenic peptide-specific TCR clones that were determined by the CD69 induction assay or staining with each EBV tetramer. | | | | | | |

### Primary T cells transduced with cloned EBV-specific TCRs killed target cells

To determine whether primary T cells transduced with the cloned EB virus-specific TCRs show cytotoxic activities to cognate antigen-presenting target cells, the inventors of the present invention prepared peripheral blood T cells from a normal donor, retrovirally transduced BRLF-1-specific Vβ5.1⁺ TCRs (Q-22, U-19, F-39) into primary T cells and compared their ability to kill T2-A24 cells, a TAP-deficient T2 cell line transfected with HLA-*240218 (Reference 12), that had been pulsed with the BRLF-1 peptide. The BRLF-1-specific MHC/p tetramer bound to 12.0%, 7.6% and 1.2% of Vβ5.1⁺ cells in the T cell population that were transduced with Q-22, U-19, and F-39, respectively (Fig. 2f). The expression level of EGFP, which was linked to the TCR gene via an internal ribosome entry site (IRES), was almost the same, indicating that the retroviral vectors had similar transduction efficiencies (Fig. 2f and Fig. 4a). The inventors of the present invention then determined the cytotoxic activities of the T cells that were transduced with the BRLF-1-specific TCRs to T2-A24 cells that had been pulsed with the BRLF-1 peptide or the EBNA3A peptide. As shown in Fig. 2g, T cells transduced with the BRLF-1-specific TCRs showed cytotoxicity to BRLF-1-pulsed T2-A24 cells, but not to EBNA3A-pulsed cells, showing that their cytotoxic activity was peptide-specific. Similarly, T cells transduced with the EBNA3A-specific TCR (E-21) showed cytotoxicity to EBNA3A-pulsed T2-A24 cells, but not to BRLF-1-pulsed cells (Fig. 5). These results show that primary T cells transduced with cloned EBV-specific TCRs have cytotoxic activities to cognate antigen-presenting target cells, indicating that the hTEC10 system may be able to identify TCR candidates for TCR gene therapy.

### Clinical application of hTEC10 system to cancer patients

To apply the hTEC10 system to cancer patients, the inventors of the present invention wanted to obtain tumor-associated antigen (TAA)-specific TCRs and examine their cytotoxicity. In previous studies, it has been shown that a patient with hepatocellular carcinoma (HCC) had alpha-fetoprotein (AFP)-specific CTL responses, and some immunogenic AFP-derived CTL epitopes have been identified (References 13 and 14). A clinical trial to determine the effectiveness of AFP-derived peptide vaccination for patients with HCC has already been performed, and several patients have shown clinical responses. In the present study, the inventors of the present invention first obtained PBLs from two HCC patients who had been treated with AFP-derived peptide vaccines and showed clinical responses. The clinical courses of these patients are shown in Fig. 6a and 6b. The first patient (patient 1), who was infected with hepatitis B virus (HBV) and had a large HCC tumor with vascular invasion to the portal vein, was vaccinated with the AFP₃₅₇ and AFP₄₀₃ peptides biweekly for 72 weeks. After vaccination, the elevated serum AFP value was normalized, and the size of the HCC decreased and eventually disappeared, as evaluated by magnetic resonance imaging (MRI) (Fig. 6a). The patient showed a complete response (CR). The second patient (patient 2), who was infected with HBV and had multiple metastatic lesions of HCC in the abdominal wall and lung, was vaccinated with the AFP₃₅₇ and AFP₄₀₃ peptides biweekly for 88 weeks. After vaccination, the elevated serum AFP value was decreased, and the metastatic lesions of HCC in the abdominal wall disappeared, as evaluated by computed tomography (CT) (Fig. 6b). The size and number of lesions from the lung metastasis did not change over the 88 weeks of treatment. This patient showed stable disease (SD).

The inventors of the present invention then incubated the PBLs, which were obtained during the treatment, with AFP-derived peptides for three weeks to expand the AFP-specific CD8⁺ T cells *in vitro.* The inventors of the present invention subsequently examined the HLA-A*2402/AFP peptide tetramer-positive CD8⁺ T cells using FACS. As shown in Fig. 6c, 1.5 and 2.6% of CD8⁺ T cells were positive with MHC/p tetramer staining in patient 1 and 2, respectively. The inventors of the present invention then sorted the single HLA-A*2402/AFP peptide tetramer-positive CD8⁺ T cells using FACS, amplified the TCR cDNA and analyzed their sequences. The inventors of the present invention obtained 73 and 126 AFP-specific TCRs from patient 1 and patient 2, respectively. The sequence analysis revealed that the hTEC10 system obtained three and four T cell clones from patient 1 and patient 2, respectively (Fig. 6d), suggesting that peptide vaccination induced the oligoclonal expansion of AFP-specific T cells in these patients. Alternatively, it is conceivable that the *in vitro* culture resulted in the expansion of oligoclonal AFP-specific T cells. It is of note that the hTEC10 system could clone TCRs from very rare antigen-specific T cells, as found in the case of EBV-specific minor clones (Fig. 6d). The inventors of the present invention then prepared peripheral blood T cells from a healthy donor, retrovirally transduced three of the obtained AFP-specific TCRs into primary T cells and analyzed the binding of the HLA-A*2402/AFP peptide tetramer using FACS. 16 to 33% of T cells were transfected with the TCRs (Fig. 4), and a range of 0.5 to 2.1 % of the total CD8⁺ cells bound the HLA-A*2402/AFP peptide (Fig. 6e). The inventors of the present invention then determined the cytotoxic activities of the transduced T cells to C1R-A24 cells pulsed with the AFP peptide. As shown in Fig. 6f, T cells transduced with TCRs showed significant cytotoxicity to the AFP peptide-pulsed C1R-A24 cells, but not to unpulsed cells, showing that the cytotoxic activity was peptide-specific. These results show that the hHTEC10 system is capable of cloning functional TAA-specific TCRs from cancer patients.

### Improvement of the hTEC10 system

The inventors of the present invention have shown the rapid, direct cloning of antigen-specific TCRs using MHC/p tetramers. However, the availability of MHC/p tetramers is limited. Thus, the inventors of the present invention tried to establish a novel system to clone TCR cDNAs from cytokine-secreting T cells after *in vitro* peptide stimulation. The inventors of the present invention obtained PBLs from healthy EBV latent donors and incubated them with the BRLF-1 peptide to expand BRLF-1 -specific CD8⁺ T cells *in vitro.* After *in vitro* culture, PBLs were re-stimulated in the presence of an anti-CD28 antibody with or without the BRLF-1 peptide. IFN-γ-secreting cells were stained using an IFN-γ secretion assay kit. As shown in Fig. 7a, 0.61% of CD8⁺ T cells were IFN-γ-positive. The inventors of the present invention then sorted the single T cells with FACS, amplified the TCR cDNAs and analyzed their sequences. The inventors of the present invention obtained 12 TCRs and compared their repertoires with those obtained from the MHC/p tetramer staining method. As a result, 83% of the repertoires of TCR obtained on the basis of secretion of cytokine were identical to the repertoires of TCR obtained by the MHC/p tetramer staining method (Fig. 7b), suggesting that the system of the present invention can be used to detect antigen-specific T cells without using MHC tetramers.

### [Discussion]

In this study, the inventors of the present invention established a rapid and direct cloning and functional evaluation system of TCR cDNA derived from single antigen-specific human T cells (hTEC10 system, Fig. 1) for analyzing antigen-specific TCR repertoires and providing prospective candidate TCRs for TCR gene therapy of cancer. The hTEC10 system is innovative for the following reasons:
1) This system enables the inventors of the present invention to obtain TCRs within 10 days, while the conventional TCR cloning method requires several months.
2) The inventors of the present invention can obtain unbiased TCR α/β pairs simultaneously from single T cells using this system, while the conventional system results in biased TCR repertoires due to the culture conditions. In this context, the hTEC10 system may identify TCR cDNAs from both major and very rare antigen-specific T cell clones (Fig. 2b and Fig. 6d).
3) This system allows us to efficiently analyze the antigen-specificity and function of the obtained TCRs.

First, the inventors of the present invention attempted to apply the hTEC10 system of the present invention for the analysis of the human T cell repertoire of healthy patients with latent infectious diseases. The inventors of the present invention obtained 379 EBV-specific TCR α/β cDNA pairs derived from 10 healthy donors and analyzed their repertoires. In agreement with previous reports (References 10 and 11), the repertoires of the EB virus-specific TCRs were highly restricted (Fig. 2b). Interestingly, the frequency of EBV-specific CD8⁺ T cells in PBLs was inversely correlated with the size of the repertoires (Fig. 2c), indicating that a few selected T cell clones regulate EBV latency.

Second, to determine prospective candidate TCRs for gene therapy of cancer, the inventors of the present invention used the hTEC10 system to analyze PBLs derived from HCC patients who had been successfully treated with AFP peptide vaccination. The inventors of the present invention obtained 73 and 126 TCR cDNA pairs from the AFP-specific CD8⁺ T cells from patient 1 and patient 2, respectively, which were categorized into seven distinct TCR repertoires. Three of them showed potent antigen-specific cytotoxicity (Fig. 5f), suggesting that high quality AFP-specific CTLs exist in HCC patients in which the AFP peptide vaccination is effective.

Third, the AFP-specific T cell clones obtained from patient 1 can be categorized into three subgroups (Fig. 6d). Out of the 73 HLA-A*2402/AFP peptide tetramer-positive T cells that were sorted from the patient 1 PBLs, 68 cells expressed the same TCR repertoire as AFP1-2, one T cell expressed AFP1-14 and four T cells expressed the same TCR repertoire as AFP 1-16. As shown in Fig. 6f, the AFP1-14 TCR conferred the highest CTL activity on T cells among the three TCRs. This result shows that the TCR that is expressed on the major population of cells does not always confer good CTL activity. In this context, the capacity of the hTEC10 system to clone a large number of antigen-specific TCR clones increases the opportunity to obtain prospective TCR candidates for TCR gene therapy.

Finally, because the production of multimeric MHC/peptide complexes, especially the MHC class II/peptide complex multimer, is still difficult, the inventors of the present invention tried to apply the hTEC10 system to detect and retrieve TCR α/β cDNA pairs from cytokine-secreting CD8⁺ T cells that were stimulated with a specific peptide. As shown in Fig. 7, the sequences of 12 TCRs obtained from IFN-γ-secreting cells that were stimulated with a specific peptide corresponded with the sequences recovered from the MHC/peptide tetramer staining of cells from the same donor. Taken together with the CD69 induction assay results shown in Fig. 2e, the inventors of the present invention can use this system to collect cytokine-secreting CD8⁺ T cells stimulated with specific peptides and obtain antigen-specific TCR without the need to stain with an MHC/peptide multimer.

In conclusion, the inventors of the present invention have, for the first time to the knowledge of the inventors of the present invention, established a rapid and efficient cloning system that can directly retrieve TCR α/β cDNA pairs from single human T cells and evaluate their functional properties within 10 days. Using this system, the inventors of the present invention demonstrated a repertoire analysis of EBV-specific CTLs. The inventors of the present invention also showed that candidate HCC-specific TCRs can be obtained from HCC patients who had been successfully treated with AFP peptide vaccination. The system of the present invention may facilitate TCR repertoire analysis and TCR gene therapy.

### Example 2

### Analysis of amplification ratio of an antigen-specific TCR α/β cDNA pair

(1) Human PBLs were incubated as a population for two days in the presence of a stimulant (IL-2 and PHA, or IL-7 and PHA). Then, CD3-positive T cells were each sorted into a tube with a cell sorter, and subjected to RT-PCR. A sample of 10 µl obtained from each tube was subjected to electrophoresis using 1% agarose gel, and stained with ethidium bromide (EtBr). The results are shown in Fig. 8. The antigen-specific TCR α/β cDNA pair amplification ratios observed after culture for two days in the presence of the stimulant were 30/30 (100%) when the cells were stimulated with Il-2 and PHA, and 30/30 (100%) when the cells were stimulated with IL-7/PHA. When the cells were cultured only with the medium (no stimulation) for two days, the amplification ratio was 17/30 (56.7%).
(2) The cells were stimulated in the same manner as that of (1), but stimulated with IL-7 alone, or IL-2 and PHA, and the influence on the amplification ratio was observed. The results are shown in Fig. 9. The antigen-specific TCR α/β cDNA pair amplification ratios obtained after culture for 2 days were 53.3% when the cells were cultured only with the medium (no stimulation), 93.3% when the cells were stimulated with Il-2 and PHA, and 66.6% when the cells were stimulated with IL-7 alone.
(3) Amplification ratios obtained with CHX and PHA/CHX. The experiment was carried out in the same manner as that used for the experiment of which results are shown in Fig. 8, except that human CD3-positive T cells were used, and the culture time in the presence of the stimulant was 12 hours. The antigen-specific TCR α/β cDNA pair amplification ratios obtained after culture for 12 hours were 46.6% when the cells were cultured only with the medium (no stimulation), 66.7% when the cells were stimulated with CHX alone, and 93.3% when the cells were stimulated with PMA/CHX.

### [References cited in the examples]

Reference 9: Kuzushima, K. et al., Tetramer-assisted identification and characterization of epitopes recognized by HLA A*2402-restricted Epstein-Barr virus-specific CD8+ T cells, Blood, 101, 1460-1468 (2003)
Reference 10: Lim, A. et al., Frequent contribution of T cell clonotypes with public TCR features to the chronic response against a dominant EBV-derived epitope: application to direct detection of their molecular imprint on the human peripheral T cell repertoire, J. Immunol., 165, 2001-2011 (2000)
Reference 11: Argaet, V.P. et al., Dominant selection of an invariant T cell antigen receptor in response to persistent infection by Epstein-Barr virus, J. Exp. Med., 180, 2335-2340 (1994)
Reference 12: Miyahara, Y. et al., Determination of cellularly processed HLA-A2402-restricted novel CTL epitopes derived from two cancer germ line genes, MAGE-A4 and SAGE, Clin. Cancer Res., 11, 5581-5589 (2005)
Reference 13: Mizukoshi, E., Nakamoto, Y., Tsuji, H., Yamashita, T. & Kaneko, S., Identification of alpha-fetoprotein-derived peptides recognized by cytotoxic T lymphocytes in HLA-A24+ patients with hepatocellular carcinoma, Int. J. Cancer, 118, 1194-1204 (2006)
Reference 14: Mizukoshi, E. et al., Comparative analysis of various tumor-associated antigen-specific t-cell responses in patients with hepatocellular carcinoma, Hepatology, 53, 1206-1216 (2011)
Reference 15: Giudicelli, V., Chaume D. & Lefranc, M.P., TMGT/V-QUEST, an integrated software program for immunoglobulin and T cell receptor V-J and V-D-J rearrangement analysis, Nucleic Acids Res., 32, W435-440 (2004)
Reference 16: Ryan, M.D., King, A.M. & Thomas, G.P., Cleavage of foot-and-mouth disease virus polyprotein is mediated by residues located within a 19 amino acid sequence, J. Gen. Virol., 72 (Pt 11), 2727-2732 (1991)

### Sequence listing free text

SEQ ID NO: 1: Peptide vaccine AFP₃₅₇
SEQ ID NO: 2: Peptide vaccine AFP₄₀₃
SEQ ID NO: 3: EBV peptide BRLF-1 198-206
SEQ ID NO: 4: EBV peptide BMLF-1 320-328
SEQ ID NO: 5: EBV peptide LMP2 222-230
SEQ ID NO: 6: EBV peptide EBNA3A 246-254
SEQ ID NO: 7: EBV peptide EBNA3B 217-225
SEQ ID NO: 8: PCR primer alpha-RT
SEQ ID NO: 9: PCR primer beta1-RT
SEQ ID NO: 10: PCR primer beta2-RT
SEQ ID NO: 11: PCR primer AP-1
SEQ ID NO: 12: PCR primer alpha-1st
SEQ ID NO: 13: PCR primer beta1-1st
SEQ ID NO: 14: PCR primer beta2-1st
SEQ ID NO: 15: Nested PCR primer AP-2
SEQ ID NO: 16: Nested PCR primer alpha-Nest
SEQ ID NO: 17: Nested PCR primer beta-Nest

## Claims

1. A method for producing a gene of T cell receptor (TCR) specific to an antigen A, *in-vitro* which comprises:
1) the step of stimulating a group of T cells including a T cell specific to an antigen A under a condition effective for amplification of a TCR gene;
2) the step of identifying a T cell specific to an antigen A among the group of T cells including a T cell specific to the antigen A, and sorting one T cell specific to the antigen A into a vessel; and
3) the step of subjecting the one activated T cell specific to the antigen A in the vessel to PCR to amplify a gene of TCR specific to the antigen A wherein the condition effective for amplification of a TCR gene mentioned in the step 1) consists of maintaining the group of cells in the presence of interleukin 2(IL-2) and phytohemagglutinin (PHA), or interleukin 7 (IL-7) and phytohemagglutinin (PHA);or
phorbol 12-myristate 13-acetate (PMA) and cycloheximide (CHX), and the steps 1), 2), and 3) are performed in this order,
step 2) is performed by flow cytometry or immunospot array assay on a chip (ISAAC) method,
wherein the step 2)
(i) is a step of sorting the cell by flow cytometry using a multimer of a complex of a major histocompatibility complex (MHC) molecule and an antigen A-derived antigen peptide (p) (MHC/p tetramer), and anti-CD8 antibody,
(ii) is a step of sorting the cell by flow cytometry using an anti-interferon γ (IFN-γ) antibody, and anti-CD8 antibody,
or
(iii) is a step using a microwell array having a plurality of wells of such a size that only one T-cell is contained in each well on one main surface of a substrate.

2. The production method according to claim 1, which further comprises:
4) the step of introducing the obtained TCR gene into a cell of a T cell strain not expressing TCR, allowing expression of the TCR, and verifying antigenic specificity of the expressed TCR.

3. The production method according to claim 1 or 2, wherein all the steps are performed within ten days.

4. A method for producing a recombinant T cell *in-vitro,* which comprises the steps defined in any one of claims 1 to 3, and further comprises a step of introducing the obtained gene of TCR specific to an antigen A into another T cell to obtain a recombinant T cell specific to the antigen A.

5. The production method according to claim 4, wherein the antigen A is an antigen relevant to a disease or condition treatable by TCR gene therapy, and the other T cell is derived from a subject with the treatable disease or condition.

6. The production method according to any one of claims 1 to 5, wherein the antigen A is a cancer-associated antigen, and a cancer-specific TCR gene or a cancer-specific recombinant T cell is produced.

7. The method according to any one of claims 1 to 3, which is performed for analysis of TCR repertoires in a subject with a cancer or an infectious disease.

## Patentansprüche

1. In-vitro-Verfahren zur Herstellung eines Gens eines für ein Antigen A spezifischen T-Zell-Rezeptors (TCR), umfassend:
1) den Schritt des Stimulierens einer Gruppe von T-Zellen, die eine für ein Antigen A spezifische T-Zelle umfassen, unter Bedingungen, die eine Amplifikation eines TCR-Gens bewirken;
2) den Schritt des Identifizierens einer für ein Antigen A spezifischen T-Zelle innerhalb der Gruppe von T-Zellen, die eine für ein Antigen A spezifische T-Zelle umfassen, und des Aussortierens einer einzigen für das Antigen A spezifischen T-Zelle in ein Gefäß; und
3) den Schritt des Durchführens einer PCR mit der einen aktivierten, für das Antigen A spezifischen T-Zelle in dem Gefäß, um ein Gen eines für das Antigen A spezifischen TCR zu amplifizieren;
wobei die in Schritt 1) erwähnten Bedingungen, die eine Amplifikation eines TCR-Gens bewirken, darin bestehen, die Gruppe von Zellen in Gegenwart von
Interleukin 2 (IL-2) und Phytohämagglutinin (PHA) oder
Interleukin 7 (IL-7) und Phytohämagglutinin (PHA) oder
Phorbol-12-myristat-13-acetat (PMA) und Cycloheximid (CHX)
zu halten,
und die Schritte 1), 2) und 3) in dieser Reihenfolge durchgeführt werden;
Schritt 2) mittels Durchflusscytometrie oder des Verfahrens des Immunspot-Array-Assay auf einem Chip (ISAAC) durchgeführt wird;
wobei der Schritt 2)
(i) ein Schritt des Aussortierens der Zelle mittels Durchflusscytometrie unter Verwendung eines Multimers aus einem Komplex aus einem Haupthistokompatibilitätskomplex(MHC)-Molekül und einem von Antigen A abgeleiteten Antigenpeptid (p) (MHC/p-Tetramer) und eines Anti-CD8-Antikörpers ist;
(ii) ein Schritt des Aussortierens der Zelle mittels Durchflusscytometrie unter Verwendung eines Anti-Interferon-γ(IFN-γ)-Antikörpers und eines Anti-CD8-Antikörpers ist;
oder
(iii) ein Schritt des Verwendens eines Mikrowell-Arrays ist, das auf einer Hauptfläche eines Substrats eine Vielzahl von Wells einer solchen Größe aufweist, dass in jedem Well nur eine einzige T-Zelle enthalten ist.

2. Herstellungsverfahren gemäß Anspruch 1, das weiterhin umfasst:
4) den Schritt des Einführens des erhaltenen TCR-Gens in eine Zelle eines T-Zell-Stamms, die kein TCR exprimiert, was eine Expression des TCR ermöglicht, und des Überprüfens der Antigenspezifität des exprimierten TCR.

3. Herstellungsverfahren gemäß Anspruch 1 oder 2, wobei alle Schritte innerhalb von zehn Tagen durchgeführt werden.

4. In-vitro-Verfahren zur Herstellung einer rekombinanten T-Zelle, das die in einem der Ansprüche 1 bis 3 definierten Schritte umfasst und weiterhin einen Schritt des Einführens des erhaltenen TCR-Gens, das spezifisch für ein Antigen A ist, in eine andere T-Zelle umfasst, wobei man eine für das Antigen A spezifische rekombinante T-Zelle erhält.

5. Herstellungsverfahren gemäß Anspruch 4, wobei das Antigen A ein Antigen ist, das für eine Krankheit oder einen Zustand, die oder der durch TCR-Gentherapie behandelbar ist, relevant ist, und die andere T-Zelle von einem Patienten mit der behandelbaren Krankheit oder dem behandelbaren Zustand stammt.

6. Herstellungsverfahren gemäß einem der Ansprüche 1 bis 5, wobei das Antigen A ein krebsassoziiertes Antigen ist und ein krebsspezifisches TCR-Gen oder eine krebsspezifische rekombinante T-Zelle hergestellt wird.

7. Verfahren gemäß einem der Ansprüche 1 bis 3, das zur Analyse der TCR-Repertoires bei einem Patienten mit einer Krebs- oder Infektionserkrankung durchgeführt wird.

## Revendications

1. Procédé de production d'un gène d'un récepteur des cellules T (TCR) spécifique d'un antigène A, *in vitro* qui comprend :
1) l'étape de stimulation d'un groupe de cellules T incluant une cellule T spécifique d'un antigène A dans une condition efficace pour amplification d'un gène TCR ;
2) l'étape d'identification d'une cellule T spécifique d'un antigène A parmi le groupe de cellules T incluant une cellule T spécifique de l'antigène A, et le tri d'une cellule T spécifique de l'antigène A dans une cuve ; et
3) l'étape de soumission de la cellule T activée spécifique de l'antigène A dans la cuve à une PCR pour amplifier un gène TCR spécifique de l'antigène A dans lequel la condition efficace pour amplification d'un gène TCR mentionné dans l'étape 1) consiste à maintenir le groupe de cellules T en présence d'interleukine 2(IL-2) et de phytohémagglutinine (PHA), ou d'interleukine 7(IL-7) et de phytohémagglutinine (PHA) ; ou
de 12-myristate 13-acétate de phorbol (PMA) et de cycloheximide (CHX), et les étapes 1), 2), et 3) sont réalisées dans cet ordre,
l'étape 2) est réalisée par un procédé de cytométrie en flux ou essai sur réseau d'immunoempreinte sur puce (ISAAC),
dans lequel l'étape 2)
(i) est une étape de tri des cellules par cytométrie en flux utilisant un multimère d'un complexe d'une molécule du complexe majeur d'histocompatibilité (CMH) et d'un peptide d'antigène dérivé d'antigène A (p) (tétramère CMH/p), et d'un anticorps anti-CD8,
(ii) est une étape de tri des cellules par cytométrie en flux utilisant un anticorps anti-interféron γ (IFN-γ), et un anticorps anti-CD8, ou
(iii) est une étape utilisant un réseau de micro-puits comportant une pluralité de puits d'une taille telle que seule une cellule T soit contenue dans chaque puits sur une surface principale d'un substrat.

2. Procédé de production selon la revendication 1, qui comprend en outre :
4) l'étape d'introduction du gène TCR obtenu dans une cellule d'une souche de cellules T n'exprimant pas TCR, permettant l'expression du TCR, et vérifiant la spécificité antigénique du TCR exprimé.

3. Procédé de production selon la revendication 1 ou 2, dans lequel toutes les étapes sont réalisées en l'espace de dix jours.

4. Procédé de production d'une cellule T recombinante *in vitro,* qui comprend les étapes définies dans l'une quelconque des revendications 1 à 3, et comprend en outre une étape d'introduction du gène obtenu de TCR spécifique d'un antigène A dans une autre cellule T pour obtenir une cellule T recombinante spécifique de l'antigène A.

5. Procédé de production selon la revendication 4, dans lequel l'antigène A est un antigène propre à une maladie ou une affection pouvant être traitée par thérapie génique TCR, et l'autre cellule T est dérivée d'un sujet atteint de la maladie ou affection pouvant être traitée.

6. Procédé de production selon l'une quelconque des revendications 1 à 5, dans lequel l'antigène A est un antigène associé au cancer, et un gène TCR spécifique du cancer ou une cellule T recombinante spécifique du cancer est produit(e).

7. Procédé selon l'une quelconque des revendications 1 à 3, qui est réalisé pour analyse de répertoires de TCR chez un sujet atteint d'un cancer ou d'une maladie infectieuse.
